# EUROPEAN PATENT APPLICATION

(11) **EP 2 436 273 A1**
(43) Date of publication of application: **04.04.2012**
(21) Application number: 11196119.9
(22) Date of filing: 04.04.2007
(51) Int. Cl.: A23L 1/035, A61K 9/107, A23D 7/00, B01J 13/02

(54) **Emulsions and microcapsules with substances having low interfacial tension, methods of making and using thereof**

(30) Priority: 07.04.2006 US 79012906 P; 05.06.2006 US 81102406 P; 11.08.2006 US 83705006 P; 10.01.2007 US 87975907 P; 10.01.2007 US 87963607 P
(62) Divisional of application: 07754635.6
(71) Applicant: Ocean Nutrition Canada Limited, Dartmouth Nova Scotia B2Y 4T6 (CA)
(72) Inventor: Jin, Yulai, Halifax Nova Scotia B3L 4P7 (CA); Zhang, Wei, Halifax Nova Scotia B3M 4Y5 (CA); Barrow, Colin James, Halifax Nova Scotia B3L 1A8 (CA)
(74) Representative: Campbell, Neil Boyd

(57) **Abstract**

Disclosed are emulsions and microcapsules that comprise one or more substance with a low interfacial tension. Methods of making the emulsion and microcapsules as well as method of using them are also disclosed. The emulsions contain gelatin and marine or microbial oil. The pH -value of the emulsion is 3.5 to 4.9.

## Description

### CROSS REFERENCE TO RELATED APPLICATIONS

This application claims the benefit of priority to U.S. Provisional Application Nos. 60/790,129, filed April 7, 2006; 60/811,024 filed June 5, 2006; 60/837,050, filed August 11, 2006; 60/879,759, filed January 10, 2007; and 60/879,636, filed January 10, 2007. U.S. Provisional Application Nos. 60/790,129, 60/811,024, 60/837,050, 60/879,759, and 60/879,636 are each incorporated by reference herein in their entireties.

### BACKGROUND

Emulsions are generally taken to mean heterogeneous systems that comprise two liquids. The two liquids, usually referred to as phases, can be immiscible or miscible to a limited extent. In an emulsion, one of the two liquids (called the dispersed phase) is dispersed in the form of very fine droplets in the other liquid (called the continuous phase). When the two liquids are water and oil, and oil droplets are very finely dispersed in water, this is called an oil-in-water emulsion (O/W emulsion). Uses of such emulsions are well known in the chemical arts, including the pharmaceutical, specialty chemical and agricultural industries. In agriculture, emulsions provide formulations vehicles for delivery of herbicides, insecticides, fungicides, bactericides and fertilizers. Non-agricultural uses include formulations of dyes, inks, pharmaceuticals, flavoring agents, and fragrances. Emulsions are also important in the preparation of microcapsules.

There are a number of factors that affect how an emulsion is prepared and the emulsion's stability, for example, interfacial tension, viscosity, relative density, and temperature. Interfacial tension is the force which acts on an imaginary line one meter in length at the interface between two phases. The physical unit for this interfacial tension is conventionally calculated from the force/length relationship and is usually expressed in dynes/cm (dynes per centimeter, which is equivalent to millinewtons per meter).

The interfacial tension between an oil and water can typically be high (*e*.*g*., about 20 to 30 dynes/cm). High interfacial tensions between the oil and water promote coalescence and creaming. Emulsifying agents such as surfactants and certain polymers that are added to the system can adsorb at the oil/water interface and reduce the interfacial tension, facilitating the formation and stabilization of an emulsion. When preparing an emulsion by mechanical shear, a low interfacial tension between the two phases is favorable for droplet formation due to lower surface free energy. If the interfacial tension is too high, the droplets formed will not be stable enough and the interface (*i*.*e*., the droplet film) will break up because of its instability with high free energy. In addition to many other factors, appropriately low interfacial tension is therefore important for emulsification and emulsion stability.

However, a too small interfacial tension can also be detrimental to the droplet stability (*i*.*e*., it can also cause coalescence of the emulsion droplets). While low interfacial tension favors droplet formation, the low surface free energy also allows the droplet films to rupture easily, leading to coalescence. As noted, the problem of high interfacial tension can be readily resolved by adding, various types and amounts of surface active materials. However, the droplet coalescence caused by low interfacial tension is a problem without an easy solution.

In light of the above, what are needed in the art are emulsions and methods for their preparation where the dispersed phase has a low interfacial tension. Methods of using such emulsions, for example to prepare microcapsules, are also needed. The compositions and methods disclosed herein meet these and other needs.

### SUMMARY

In accordance with the purposes of the disclosed materials, compounds, compositions, articles, and methods, as embodied and broadly described herein, the disclosed subject matter, in one aspect, relates to compositions and methods for preparing and using such compositions. In a further aspect, the disclosed subject matter relates to emulsions that comprise a first polymer component and a loading substance. In a still further aspect, the disclosed subject matter relates to microcapsules that comprise an agglomeration of primary microcapsules and a loading substance encapsulated within the primary microcapsules. The agglomeration of primary microcapsules is encapsulated by one or more outer shells. Also disclosed are methods of making and using the disclosed emulsions and microcapsules.

Additional advantages will be set forth in part in the description that follows, and in part will be obvious from the description, or may be learned by practice of the aspects described below. The advantages described below will be realized and attained by means of the elements and combinations particularly pointed out in the appended claims. It is to be understood that both the foregoing general description and the following detailed description are exemplary and explanatory only and are not restrictive.

### DETAILED DESCRIPTION

The materials, compounds, compositions, and methods described herein may be understood more readily by reference to the following detailed description of specific aspects of the disclosed subject matter and the Examples included therein.

Before the present materials, compounds, compositions, and methods are disclosed and described, it is to be understood that the aspects described below are not limited to specific synthetic methods or specific reagents, as such may, of course, vary. It is also to be understood that the terminology used herein is for the purpose of describing particular aspects only and is not intended to be limiting.

Also, throughout this specification, various publications are referenced. The disclosures of these publications in their entireties are hereby incorporated by reference into this application in order to more fully describe the state of the art to which the disclosed matter pertains. The references disclosed are also individually and specifically incorporated by reference herein for the material contained in them that is discussed in the sentence in which the reference is relied upon.

### General Definitions

In this specification and in the claims that follow, reference will be made to a number of terms, which shall be defined to have the following meanings:

Throughout the description and claims of this specification the word "comprise" and other forms of the word, such as "comprising" and "comprises," means including but not limited to, and is not intended to exclude, for example, other additives, components, integers, or steps.

As used in the description and the appended claims, the singular forms "a," "an," and "the" include plural referents unless the context clearly dictates otherwise. Thus, for example, reference to "a compound" includes mixtures of two or more such compounds, reference to "an acid" includes mixtures of two or more such acids, reference to "the salt" includes mixtures of two or more such salts, and the like.

"Optional" or "optionally" means that the subsequently described event or circumstance can or cannot occur, and that the description includes instances where the event or circumstance occurs and instances where it does not.

Ranges can be expressed herein as from "about" one particular value, and/or to "about" another particular value. When such a range is expressed, another aspect includes from the one particular value and/or to the other particular value. Similarly, when values are expressed as approximations, by use of the antecedent "about," it will be understood that the particular value forms another aspect. It will be further understood that the endpoints of each of the ranges are significant both in relation to the other endpoint, and independently of the other endpoint. It is also understood that there are a number of values disclosed herein, and that each value is also herein disclosed as "about" that particular value in addition to the value itself. For example, if the value "10" is disclosed, then "about 10" is also disclosed. It is also understood that when a value is disclosed that "less than or equal to" the value, "greater than or equal to the value," and possible ranges between values are also disclosed, as appropriately understood by the skilled artisan. For example, if the value "10" is disclosed, then "less than or equal to 10" as well as "greater than or equal to 10" is also disclosed. It is also understood that throughout the application data is provided in a number of different formats and that this data represents endpoints and starting points and ranges for any combination of the data points. For example, if a particular data point "10" and a particular data point "15" are disclosed, it is understood that greater than, greater than or equal to, less than, less than or equal to, and equal to 10 and 15 are considered disclosed as well as between 10 and 15. It is also understood that each unit between two particular units are also disclosed. For example, if 10 and 15 are disclosed, then 11,12,13, and 14 are also disclosed.

References in the specification and concluding claims to parts by weight of a particular component in a composition denotes the weight relationship between the component and any other components in the composition for which a part by weight is expressed. Thus, in a compound containing 2 parts by weight of component X and 5 parts by weight component Y, X and Y are present at a weight ratio of 2:5, and are present in such ratio regardless of whether additional components are contained in the compound.

A weight percent (wt.%) of a component, unless specifically stated to the contrary, is based on the total weight of the formulation or composition in which the component is included.

"Subject," as used herein, means an individual. In one aspect, the subject is a mammal such as a primate, and, in another aspect, the subject is a human. The term "subject" also includes domesticated animals (*e*.*g*., cats, dogs, etc.), livestock (*e*.*g*., cattle, horses, pigs, sheep, goats, etc.), and laboratory animals (*e*.*g*., mouse, rabbit, rat, guinea pig, fruit fly, etc.).

"Emulsions" is used herein to mean any heterogenous system that contains a disperse phase and continuous phase. The term is not intended to be limited by the particular size of the dispersed phase, *e*.*g*., "emulsion" includes macroemulsions, microemulsions, and nanoemulsions.

Reference will now be made in detail to specific aspects of the disclosed materials, compounds, compositions, articles, and methods, examples of which are illustrated in the accompanying Examples.

### Materials and Compositions

Disclosed herein are materials, compounds, compositions, and components that can be used for, can be used in conjunction with, can be used in preparation for, or are products of the disclosed methods and compositions. These and other materials are disclosed herein, and it is understood that when combinations, subsets, interactions, groups, etc, of these materials are disclosed that while specific reference of each various individual and collective combinations and permutation of these compounds may not be explicitly disclosed, each is specifically contemplated and described herein. For example, if a compound is disclosed and a number of modifications that can be made to a number of components or residues of the compound are discussed, each and every combination and permutation that are possible are specifically contemplated unless specifically indicated to the contrary. Thus, if a class of components A, B, and C are disclosed as well as a class of components D, E, and F and an example of a combination composition A-D is disclosed, then even if each is not individually recited, each is individually and collectively contemplated. Thus, in this example, each of the combinations A-E, A-F, B-D, B-E, B-F, C-D, C-E, and C-F are specifically contemplated and should be considered disclosed from disclosure of A, B, and C; D, E, and F; and the example combination A-D. Likewise, any subset or combination of these is also specifically contemplated and disclosed. Thus, for example, the sub-group of A-E, B-F, and C-E are specifically contemplated and should be considered disclosed from disclosure of A, B, and C; D, E, and F; and the example combination A-D. This concept applies to all aspects of this disclosure including, but not limited to, steps in methods of making and using the disclosed compositions. Thus, if there are a variety of additional steps that can be performed it is understood that each of these additional steps can be performed with any specific aspect or combination of aspects of the disclosed methods, and that each such combination is specifically contemplated and should be considered disclosed.

### Emulsions

Disclosed herein, in one aspect, are emulsions that comprise a first polymer component and a loading substance. In the disclosed emulsions, the loading substance can comprise a long chain polyunsaturated fatty acid and have an interfacial tension of less than about 20 dynes/cm (*e*.*g*., less than about 15 dynes/cm). In a specific example, the disclosed emulsions can contain as a loading substance a microbial oil with an interfacial tension of less than about 15, less than about 10, less than about 5, less than about 3, less than about 2, less than about 1, or less than about 0.5 dynes/cm. This interfacial tension can be measured using the aqueous materials used in the disclosed processes.

### pH

The emulsions can also have a pH of greater than about 6.0 or less than about 5.0. In specific examples, an emulsion's pH can be from about 3.5 to about 4.9, or from about 9.0 to about 11.0. In other specific examples, the disclosed emulsions can have a pH of about 0.1, 0.2, 0.3, 0.4, 0.5, 0.6, 0.7, 0.8, 0.9, 1.0, 1.1, 1.2, 1.3,1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, 8.0, 8.1, 8.2, 8.3, 8.4, 8.5, 8.6, 8.7, 8.8, 8.9, 9.0, 9.1, 9.2, 9.3, 9.4, 9.5, 9.6, 9.7, 9.8, 9.9, 10.0, 10.1, 10.2, 10.3, 10.4, 10.5, 10.6, 10.7, 10.8, 10.9, 11.0, 11.1, 11.2, 11.3, 11.4, 11.5, 11.6, 11.7, 11.8, 11.9, 12.0, 12.1, 12.2, 12.3, 12.4, 12.5, 12.6, 12.7, 12.8, 12.9, 13.0, 13.1, 13.2, 13.3, 13.4, 13.5, 13.6, 13.7, 13.8, 13.9, or 14.0, where any of the stated values can form an upper or lower endpoint when appropriate. Such pH's can be obtained by adding acidic or basic materials to the emulsion after it is formed, during its formation, or to a mixture of first polymer component and loading substance prior to emulsification. Examples of suitable acidic and basic substances that can be used to obtain a desired emulsion pH include, but are not limited to, lithium hydroxide, sodium hydroxide, potassium hydroxide, calcium hydroxide, magnesium hydroxide, phosphoric acid, hydrochloric acid, nitric acid, or acetic acid, including mixtures thereof.

The pH of the disclosed emulsions can be performed by methods well know in the art. Such methods include the use of a pH meter, the use of a pH strip, colormetric kits, or titration.

### Size

The disclosed emulsions can have droplets of various sizes. Fore example, the disclosed emulsion can be microemulsions and/or nanoemulsions. That is, the droplets of the disclosed emulsions can be in the micrometer range (*i*.*e*., I to 1000 µm) or nanometer range (*i*.*e*., 1 to 1000 nm, typically less than about 0.1 µm). Specific examples include, but are not limited to, emulsions that have an average droplet size of less than about 1000, 750, 500, 250, 100, or 50 nm, where any of the stated values can form an upper or lower endpoint when appropriate.

The size of the droplets can be determined by methods known in the art, such as light scattering, microscopy, spectroscopically, and the like.

### First Polymer Component

In the disclosed emulsions, the first polymer component can comprise a surfactant, gelatin, polyphosphate, polysaccharide, or mixtures thereof. Further examples of suitable materials for the first polymer component include, but are not limited to, gelatin type A, gelatin type B, polyphosphate, gum arabic, alginate, chitosan, carrageenan, pectin, low methoxyl pectin, starch, modified starch, alpha-lactalbumin, beta-lactoglobumin, ovalbumin, polysorbiton, maltodextrin, cyclodextrin, cellulose; methyl cellulose, ethyl cellulose, hydropropylmethylcellulose, carboxymethylcellulose, milk protein, whey protein, soy protein, canola protein, albumin, xanthan, gellan gum, agar, kosher gelatin, non-kosher gelatin, Halal gelatin, and non-Halal gelatin, including combinations and mixtures thereof. One specific type of first polymer component that can be used in the disclosed emulsions is fish gelatin.

In many examples disclosed herein, the first polymer component can have a Bloom number of from about 0 to about 300. The Bloom number describes the gel strength formed at 10°C with a 6.67% solution gelled for 18 hours. Determining the Bloom number of a substance can be accomplished by.methods known in the art. In some specific examples the first polymer component can have a Bloom number of from about 0 to about 50, and in other examples the first polymer component can have a Bloom number of from about 51 to about 300. Still other specific examples include emulsions comprising a first polymer component having a Bloom number of about 0, about 210, about 220, or about 240. It is contemplated that the first polymer component can have a Bloom number of about 0, 5, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 195, 200, 205, 210, 215, 220, 225, 230, 235, 240, 245, 250, 255, 260, 265, 270, 275, 280, 285, 290, 295, or 300, where any of the stated values can form an upper or lower endpoint when appropriate.

### Loading Substance

In the disclosed emulsions, as well as in the microcapsules and methods disclosed herein, the loading substance can have a low interfacial tension. For example, a suitable loading substance can have an interfacial tension of less than about 20, less than about 15, less than about 11, less than about 9, less than about 7, less than about 5, less than about 3, less than about 2, less than about 1, or less than about 0.5 dynes/cm. In other examples, the loading substance can have an interfacial tension of from about 0.1 to about 20, from about 1 to about 15, from about 2 to about 9, from about 3 to about 9, from about 4 to about 9, from about 5 to about 9, from about 2 to about 7, from about 0.1 to 5, from about 0.3 to 2, or from about 0.5 to 1 dynes/cm. In still further examples, the loading substance can have an interfacial tension of about 0.1, 0.5, 1.0, 1.5, 2.0, 2.5, 3.0, 3.5, 4.0, 4.5, 5.0, 5.5, 6.0, 6.5, 7.0, 7.5, 8.0, 8.5, 9.0, 9.5, 10.0, 10.5, 11.0, 11.5, 12.0, 12.5, 13.0, 13.5, 14.0, 14.5, 15.0, 15.5, 16.0, 16.5, 17.0, 17.5, 18.0, 18.5, 19.0, 19.5, or 20.0, where any of the stated values can form an upper or lower endpoint when appropriate. In particular examples, the loading substance can be a marine oil with an interfacial tension of about 0.5, 0.6, 0.7, 0.8, 0.9, or 1.0 dynes/cm. The loading substance can also be an algal or fungal oil with an interfacial tension of about 3.0, 3.1, 3.2, 3.3, or 3.4 dynes/cm.

The interfacial tension of a loading substance can be determined by methods known in the art. For example, the interfacial tension from a loading substance to a standard gelatin solution or from a loading substance to distilled water can be determined with a Fisher Surface Tensiomat. Generally, a standard gelatin solution or distilled water can be poured into a sample vessel, which is placed on the sample table of a tensiomat. The loading substance can then be added to the sample vessel. The sample can be raised so that the ring of the tensiomat is immersed in the loading substance. The interfacial tension is the measure of downward force on the ring as it passes through the interface of the loading substance and standard gelatin solution or the interface of the loading substance and distilled water, depending on whichever experimental setup is being used.

The interfacial tension measurements disclosed herein for the loading substances refer to values determined asjust described using a standard gelatin solution (50°C) that contains 3.3% (w/w) of 240 Bloom kosher fish gelatin (*e*.*g*., from LAPI, Tuscany, Italy), 0.5% (w/w) sodium ascorbate, and 0.33% (w/w) polyphosphate solution dissolved in distilled water.

Suitable loading substances that can be present in the disclosed emulsions, as well as the disclosed microcapsules, can be any substance that is not entirely soluble in an aqueous mixture. The loading substance can be a solid, a hydrophobic liquid, or a mixture of a solid and a hydrophobic liquid. In many of the examples herein, the loading substance can comprise a long chain polyunsaturated fatty acid, specific examples of which are included below. Further, the loading substance can comprise a biologically active substance, a nutritional supplement, and/or a flavoring substance, including mixtures and combinations thereof. In other examples, the loading substance can comprise microbial oil, for example, and algal oil (*e*.*g*., oil from a dinoflagellate such as *Crypthecodinium cohnii*) or fungal oil (*e*.*g*., oil from *Thraustochytrium*, *Schizochytrium*, or a mixture thereof), and/or plant oil, including mixtures and combinations thereof.

In still other examples, the disclosed emulsions (and microcapsules) can comprise marine oil, such as natural and refined and concentrated fish oil. Examples of suitable fish oils include, but are not limited to, Atlantic fish oil, Pacific fish oil, Mediterranean fish oil, light pressed fish oil, alkaline treated fish oil, heat treated fish oil, light and heavy brown fish oil, bonito oil, pilchard oil, tuna oil, sea bass oil, halibut oil, spearfish oil, barracuda oil, cod oil, menhaden oil, sardine oil, anchovy oil, capelin oil, Atlantic cod oil, Atlantic herring oil, Atlantic mackerel oil, Atlantic menhaden oil, salmonid oil, and shark oil, including mixtures and combinations thereof Non-alkaline treated fish oil is also a suitable loading substance. Other marine oils suitable for use herein include, but are not limited to, squid oil, cuttle fish oil, octopus oil, krill oil, seal oil, whale oil, and the like, including mixtures and combinations thereof. Any marine oil and combination of marine oil can be used in the disclosed compositions and in the disclosed methods to prepare them.

### Omega-3 Fatty Acids

Many of the microbial, algal, fungal, plant, and marine oils disclosed herein contain omega-3 fatty acids. As such, certain emulsions (and microcapsules) disclosed herein can contain a loading substance that comprises an omega-3 fatty acid, an alkyl ester of an omega-3 fatty acid, a triglyceride ester of an omega-3 fatty acid, a phytosterol ester of an omega-3 fatty acid, and/or mixtures and combinations thereof

An omega-3 fatty acid is an unsaturated fatty acid that contains as its terminus CH₃-CH₂-CH+=CH—. Generally, an omega-3 fatty acid has the following formula: 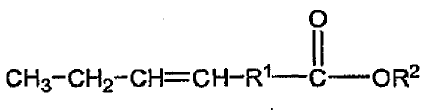 wherein R¹ is a C₃-C₄₀ alkyl or alkenyl group comprising at least one double bond and R² is H or alkyl group. The term "alkane" or "alkyl" as used herein is a saturated hydrocarbon group (*e*.*g*., methyl, ethyl, n-propyl, isopropyl, n-butyl, isobutyl, s-butyl, t-butyl, n-pentyl, isopentyl, s-pentyl, neopentyl, hexyl, heptyl, octyl, nonyl, decyl, dode cyl, tetradecyl, hexadecyl, eicosyl, tetracosyl, and the like). The term "alkene" or "alkenyl" as used herein is a hydrocarbon group containing at least one carbon-carbon double bond. Asymmetric structures such as (AB)C=C(CD) are intended to include both the E and Z isomers (*cis* and *trans*). In a further example, R¹ can be a C₅-C₃₈, C₆-C₃₆, C₈-C₃₄, C₁₀-C₃₂, C₁₂-C₃₀, C₁₄-C₂₈, C₁₆-C₂₆, or C₁₈-C₂₄ alkenyl group. In yet another example, the alkenyl group of R¹ can have from 2 to 6, from 3 to 6, from 4 to 6, or from 5 to 6 double bonds. Still further, the alkenyl group of R¹ can have from 1, 2, 3, 4, 5, or 6 double bonds, where any of the stated values can form an upper or lower endpoint as appropriate.

Specific examples of omega-3 fatty acids that are suitable loading substances include, but are not limited to, linolenic acid (18:3ω3), octadecatetraenoic acid (18:4ω3), eicosapentaenoic acid (20:3ω3) (EPA), docosahexaenoic acid (22:6ω3) (DHA), docosapentaenoic acid (22:5ω3) (DPA), including derivatives and mixtures thereof. In other specific examples the loading substance can comprise docosahexaenoic acid and/or eicosapentaenoic acid, a C₁-C₆ alkyl ester thereof, a triglyceride ester thereof, a phytosterol ester thereof, and/or a mixture thereof.

### Other Fatty Acids

Other examples of suitable loading substances that can be present in the disclosed emulsions (and microcapsules) comprise at least 8, at least 10, at least 12, at least 14, at least 16, at least 18, or at least 20 carbon atoms. In some other examples, the loading substance can contain about 8, 9, 10, 11, 12, 13, 14, 15, 16, 17, 18, 19, 20, 21, 22, 23, 24, 25, 26, 27; 28, 29, 30, 31, 32, 33, 34, 35, 36, 37, 38, 39, 40, 41, 42, 43, 44, or 45 carbon atoms, where any of the stated values can form an upper or lower endpoint when appropriate. In still other examples, the loading substance can comprise a mixture of fatty acids (including derivatives thereof) having a range of carbon atoms. For example, the loading substance can comprise from about 8 to about 40, from about 10 to about 38, from about 12 to about 36, from about 14 to about 34, from about 16 to about 32, from about 18 to about 30, or from about 20 to about 28 carbon atoms.

Some further examples of loading substances are those that contain at least one unsaturated bond (*i*.*e*., a carbon-carbon double or triple bond). For example, the loading substance can contain at least 2, at least 3, at least 4, at least 5, at least 6, at least 7, or at least 8 carbon-carbon double bonds, triple bonds, or any combination thereof. In another example, the loading substance can comprise 1, 2, 3, 4, 5, 6, 7, or 8 unsaturated bonds, where any of the stated values can form an upper or lower endpoint as appropriate.

Some specific examples of loading substances, which are unsaturated fatty acids, are shown in the following tables. Derivatives of these fatty acids are also suitable and are thus contemplated herein.

**Table 1: Examples of Monoene Acids**

| Total number of carbon atoms in the fatty acid chain | Carbon number where double bond begins. ("c" denotes a cis double bond; "t" denotes a trans double bond) |
|---|---|
| 10 | 4c |
| 12 | 4c |
| 14 | 4c and 9c |
| 16 | 3t, 4c, 5t, 6c, 6t, 9c (palmitooleic), and 11c |
| 18 | 3t, 5c, 5t, 6c (petroselinic), 6t, 9c (oleic), 10c, 11c (cis-vaccenic), 11t (vaccenic), and 13c |
| 20 | 5c, 9c (gadolenic), 11c, 13c, and 15c |
| 22 | 5c, 11c (cetoleic), 13c (erucic), and 15c |
| 24 | 15c (selacholeic, nervonic) |
| 26 | 9c, and 17c (ximenic) |
| 28 | 9c, 19c (lumequic) |
| 30 | 21c |

Unsaturated fatty acids that contain at least one pair of methylene interrupted unsaturated bonds are also suitable loading substances. By "methylene interrupted unsaturated bond" is meant that one carbon-carbon double or triple bond is separated from another carbon-carbon double or triple bond by at least one methylene group (*i*.*e*., CH₂). Specific examples of such loading substances include, but are not limited to, the n-1 family derived from 9, 12, 15-16:3; n-2 family derived from 9, 12, 15-17:3, 15:3, 17:3, 17:4, 20:4; n-3 family derived from 9, 12, 15-18:3, 15:2, 15:3, 15:4, 16:3, 16:4, 18:3 (α-linolenic), 18:4, 18:5, 20:2, 20:3, 20:4; 20:5 (EPA), 21:5,22:3,22:5 (DPA), 22:6 (DBA), 24:3, 24:4, 24:5, 24:6, 26:5, 26:6, 28:7, 30:5; n-4 family derived from 9,12-16:2, 16:2, 16:3, 18:2, 18:3; n-5 family derived from 9, 12-17:2, 15:2, 17:2, 17:3, 19:2, 19:4, 20:3, 20:4 21:4, 21:5; n-6 family derived from 9, 12-18:2, 15:2, 16:2, 18:2 (linoleic acid), 18:3 (γ-linolenic acid); 20:2, 20:3, 20:4 (arachidonic acid), 22:2, 22:3, 22:4 (adrenic acid), 22:5, 24:2, 24:4, 25:2, 26:2, 30:4; n-7 family derived from 9-16:1, 15:2, 16:2, 17:2, 18:2, 19:2; n-8 family derived from 9-17:1, 15:2, 16:2, 17:2, 18:2, 19:2; n-9 family derived from 9-18:1, 17:2, 18:2, 20:2, 20:3, 22:3, 22:4; n-11 family 19:2, and the n-12 family 20:2. In one particular specific example, the loading substance can comprise arachidonic acid.

In the above paragraph (and throughout) the compounds are identified by referring first to the "n-x family," where x is the position in the fatty acid where the first double bond begins. The numbering scheme begins at the terminal end of the fatty acid, where, for example, the terminal CH₃ group is designated position 1. In this sense, the n-3 family would be an omega-3 fatty acid, as described above. The next number identifies the total number of carbon atoms in the fatty acid. The third number, which is after the colon, designates the total number of double bonds in the fatty acid. So, for example, in the n-1 family, 16:3, refers to a 16 carbon long fatty acid with 3 double bonds, each separated by a methylene, wherein the first double bond begins at position 1, *i*.*e*., the terminal end of the fatty acid. In another example, in the n-6 family, 18:3, refers to an 18 carbon long fatty acid with 3 methylene separated double bonds beginning at position 6, *i*.*e*., the sixth carbon from the terminal end of the fatty acid, and so forth.

Further examples of loading substances that contain at least one pair of methylene interrupted unsaturated bonds are shown in Table 2.

**Table 2: Examples of Polyene Acids.**

| Total number of carbon atoms in the fatty acid chain | Carbon number where double bond begins. ("c" denotes a cis double bond; "t" denotes a trans double bond) |
|---|---|
| 18 | 5,9 |
| | 5, 11 |
| | 2t, 9, 12 |
| | 3t, 9, 12 |
| | 5t, 9, 12 |
| | 5, 9, 12 |
| | 5, 11, 14 |
| | 3t, 9, 12, 15 |
| | 5, 9, 12, 15 |
| 20 | 5, 11 |
| | 5, 13 |
| | 7, 11 |
| | 7, 13 |
| | 5, 11, 14 |
| | 7, 11, 14 |
| | 5, 11, 14, 17 |
| 22 | 5, 11 |
| | 5, 13 |
| | 7,13 |
| | 7,15 |
| | 7, 17 |
| | 9, 13 |
| | 9, 15 |

Specific examples of suitable loading substances that contain conjugated unsaturated bonds include, but are not limited to, those in Table 3. By "conjugated unsaturated bond" is meant that at least one pair of carbon-carbon double and/or triple bonds are bonded together, without a methylene (CH₂) group between them (*e*.*g*., — CH=CH-CH=CH—).

**Table 3: Examples of Conjugated Polyene Acids**

| Total number of carbon atoms in the fatty acid chain. | Carbon number where double bond begins. ("c" denotes a cis double bond; "t" denotes a trans double bond) |
|---|---|
| 10 | 2t, 4t, 6c |
| | 2c, 4t, 6t |
| | 3t, 5t, 7c |
| | 3c, 5t, 7t |
| 12 | 3, 5, 7, 9, 11 |
| 14 | 3, 5, 7, 9, 11 |
| 18 | 10t, 12t |
| | 8c, 10t, 12c (jacaric) |
| | 8t, 10t, 12c (calendic) |
| | 8t, 10t, 12t |
| | 9t, 11t, 13c (Catalpic) |
| | 9c, 11t, 13t (α-eleostearic) |
| | 9c,11t, 13c (punicic) |
| | 9t, 11t, 13t (β-eleostearic) |
| | 9c, 11t, 13t, 15c (α-parinaric) |
| | 9t, 11t, 13t, 15t (β-parinaric) |

In the above examples of suitable loading substances, derivatives of the disclosed loading substances can also be used. By "derivatives" is meant the ester of a fatty acid (*e*.*g*., methyl and ethyl esters), salts of the fatty acids (*e*.*g*., sodium and potassium salts), and triglycerides, diglycerides, and monoglyceride derivatives.

The loading substances disclosed herein can also be crude oils, semi-refined (also called alkaline refined), or refined oils from such sources disclosed herein. Still further, the disclosed compositions and methods can use oils comprising re-esterified triglycerides.

It is contemplated herein that one or more of the disclosed loading substances can be used. For example the disclosed emulsions (and microcapsules) can contain two or more different loading substances. Further, the loading substance can be present in an amount of from about 1% to about 50% by weight of the emulsion. In specific examples, the loading substance can be present in an amount of from about 1% to about 40%, from about 1% to about 30%, from about 1% to about 20%, from about 1% to about 15%, or from about 1% to about 10%.

### Specific Emulsions

The disclosed emulsions can contain any of the first polymer components and any of the loading substances disclosed herein. Some specific examples include, but are not limited to, a first polymer component that is a 240 Bloom fish gelatin, a loading substance that is microbial oil, and a pH from about 9 to about 11. In another specific example, the disclosed emulsions can have a first polymer component that is a 240 Bloom fish gelatin, a loading substance that is microbial oil, and a pH from about 3.5 to about 4.9. In yet another example, the disclosed emulsions can have a first polymer component that is a 0 Bloom fish gelatin, a loading substance that is microbial oil, and a pH from about 9 to about 11. In still another example, the disclosed emulsions can have a first polymer component that is 0 Bloom fish gelatin, a loading substance that is microbial oil, and a pH from about 3.5 to about 4.9.

The disclosed emulsions can further contain a surfactant. Such a surfactant can be used in addition to any surfactant that can be used as the first polymer component. Examples of suitable surfactants include, but are not limited to, sorbitan trioleate (Span 85), sorbitan tristearate (Span 65), sorbitan sesquioleate (Arlacel 83), glyceryl monostearate, sorbitan monooleate (Span 80), sorbitan monostearate (Span 60), sorbitan monopalmitate (Span 40), sorbitan monolaurate (Span 20), polyoxyethylene sorbitan tristearate (Tween 65), polyoxyethylene sorbitan trioleate (Tween 85), polyethylene glycol 400 monostearate, polysorbate 60 (Tween 60), polyoxyethylene monostearate, polysorbate 80 (Tween 80), polysorbate 40 (Tween 40), and polysorbate 20 (Tween 20), including mixtures and combinations thereof.

### Microcapsules

Also disclosed herein are microcapsules that comprise an agglomeration of primary microcapsules and a loading substance. Each individual primary microcapsule has a primary shell. The loading substance is encapsulated by the primary shell and the agglomeration is encapsulated by an outer shell. In the disclosed microcapsules the loading substance can be any of the loading substances disclosed herein, for example, those disclosed above for the emulsions. Some specific loading substances are disclosed herein and include, but are not limited to, long chain polyunsaturated fatty acids that have an interfacial tension of less than about 20, less than about 15, or less than about 9 dynes/cm, *e*.*g*., from 2 to about 9 dynes/cm.

The primary shell and/or outer shell of the disclosed microcapsules can comprise any of the materials disclosed above for the first polymer component of the disclosed emulsions. For example, the primary and/or outer shell can comprise a surfactant, gelatin, polyphosphate, polysaccharide, or a mixture thereof. Further examples of suitable primary shell and/or outer shell include, but are not limited to, gelatin type A, gelatin type B, polyphosphate, gum arabic, alginate, chitosan, carrageenan, pectin, low methoxyl pectin, starch, modified starch, alpha-lactalbumin, beta-lactoglobumin, ovalbumin, polysorbiton, maltodextrin, cyclodextrin, cellulose, methyl cellulose, ethyl cellulose, hydropropylmethylcellulose, carboxymethylcellulose, milk protein, whey protein, soy protein, canola protein, albumin, xanthan, gellan gum, agar, kosher gelatin, non-kosher gelatin, Halal gelatin, non-Halal gelatin, or a mixture thereof. In another example the primary shell and/or outer shell can comprise fish gelatin.

As noted above for the first polymer component of the disclosed emulsions, the primary shell and/or outer shell of the disclosed microcapsules can comprise a gelatin with a Bloom number of from about 0 to about 300, from about 0 to about 50, or from about 51 to about 300. Any of the Bloom numbers disclosed herein for the first polymer component of the disclosed emulsion, *e*.*g*., about 0, about 210, about 220, or about 240, can be used herein for the primary shell and/or outer shell of the disclosed microcapsules.

In many of the disclosed microcapsules the primary shell and/or outer shell can comprise a complex coacervate. For example, the primary shell and/or outer shell can comprise a coacervate of gelatin and polyphosphate. In other examples, the primary shell and/or outer shell can comprise a coacervate of gelatin and alginate, gelatin and pectin (*e*.*g*, low methoxyl pectin), gelatin and gum arabic, gelatin and xanthan, gelatin and whey protein, gelatin and soy protein, whey and gellan gum, whey and agar, whey and gellan gum and agar, and whey and pectin.

In the disclosed microcapsules, the average diameter of the entire agglomeration, including the outer shell, can be from about 1 µm to about 2,000 µm, from about 20 µm to about 1,000 µm, or from about 30 µm to about 80 µm. In further examples, the average diameter of the microcapsules can be about 1, 10, 20, 30, 40, 50, 60, 70, 80, 90, 200, 300, 400, 500, 600, 700, 800, 900, 1000, 1200, 1300, 1400, 1500, 1600, 1700, 1800, 1900, or 2000 µm, where any of the stated values can form an upper or lower endpoint when appropriate.

The primary microcapsules of the disclosed microcapsules can have an average diameter of from about 40 nm to about 10 µm or from about 0.1 µm to about 5 µm. In further examples, the average diameter of the primary microcapsules can be about 40 nm, 50 nm, 60 nm, 70 nm, 80 nm, 90 nm, 100 nm, 200 nm, 300 nm, 400 nm, 500 nm, 600 nm, 700 nm, 800 nm, 900 nm, 1000 nm, 2 µm, 3 µm, 4 µm, 5 µm, 6 µm, 7 µm, 8 µm, 9 µm, 10 µm, where any of the stated values can form an upper or lower endpoint when appropriate.

Particle size can be measured using any typical equipment known in the art, for example, a Coulter LS230 Particle Size Analyzer, Miami, Florida, USA.

The microcapsules disclosed herein generally have a combination of high payload and structural strength. For example, payloads of loading substance can be from 20% to 90%, 50% to 70% by weight, or 60% by weight of the microcapsules. In other examples, the disclosed microcapsules can contain about 20, 25, 30, 35, 40, 45, 50, 55, 60, 65, 70, 75, 80, 85, or 90% by weight of the microcapsule, where any of the stated values can form an upper or lower endpoint when appropriate.

In one specific example of the disclosed microcapsules, the primary shell and outer shell can comprise a coacervate of 240 Bloom fish gelatin and sodium polyphosphate, and the loading substance can be microbial oil. In another specific example, the primary shell and outer shell can comprise a coacervate of 0 Bloom fish gelatin and sodium polyphosphate, and the loading substance can be microbial oil.

It is also contemplated that one or more additional shell layers can be placed on the outer shell of the microcapsules. The techniques described in International Publication No. WO 2004/041251 A1, which is incorporated by reference in its entirety, can be used to add additional shell layers to the microcapsules.

### Antioxidants

The emulsions and microcapsules disclosed herein can also contain an antioxidant. Suitable examples of antioxidants include, but are not limited to, a phenolic compound, a plant extract, or a sulphur-containing compound. In certain examples disclosed herein the antioxidant can be ascorbic acid or a salt thereof, *e.g.*, sodium ascorbate. In other examples, the antioxidant can be vitamin E, CoQ₁₀, tocopherols, lipid soluble derivatives of more polar antioxidants such as ascobyl fatty acid esters (*e.g.*, ascobyl palmitate), plant extracts (*e.g*., rosemary, sage and oregano oils), algal extracts, and synthetic antioxidants (*e.g.*, BHT, TBHQ, ethoxyquin, alkyl gallates, hydroquinones, tocotrienols).

### Methods of Making Emulsions

Methods for preparing the emulsions disclosed herein are also described. In general, the disclosed emulsions can be prepared by providing an aqueous mixture of a first polymer component and a loading substance and emulsifying the mixture. In these methods, the loading substance can be any of the loading substances disclosed herein for the emulsions and/or microcapsules. For example, the loading substance can comprise a long chain polyunsaturated fatty acid and have an interfacial tension of less than about 15 dynes/cm. Further, the loading substance can be provided in an amount of from about 1% to about 50% by weight of the aqueous mixture. The first polymer component can also be any of the first polymer components disclosed herein for the emulsions and/or microcapsules.

In the disclosed processes for preparing emulsions, the mixture has a pH of greater than about 6.0 or less than about 5.0, for example, from about 3.5 to about 4.9 or from about 9.0 to about 11.0. Other specific pH's that are suitable in the disclosed processes are disclosed herein for the emulsions. Obtaining a pH of greater than about 6.0 can be achieved by adding a basic substance as described herein, for example, sodium hydroxide, to the mixture. Obtaining a pH of less than about 5.0 can be achieved by adding an acidic substance as described herein, for example, phosphoric acid, to the mixture. The amount of basic and/or acidic substance added in order to reach the desired pH can be determined by the skilled artisan by monitoring the pH of the mixture while adding the basic or acidic substance. Further, the mixture can have a pH of greater than about 6.0 or less than about 5.0 before, during, and/or after emulsification.

Emulsifying the mixture can be accomplished by methods and apparatus known in the art, *e.g.*, homogenization and high pressure/high shear pumps. For example, emulsification can take place by emulsifying at from about 1,000 to about 15,000 rpm. The emulsification step can be monitored by removing a sample of the mixture and analyzing it under such methods as microscopy, light scattering, turbidity, etc. Generally, emulsification can be performed until an average droplet size of less than about 1,000, 750, 500, 100, or 10 nm is obtained. Not wishing to be bound by theory, by varying the emulsification speed it is possible to produce single or multicore microcapsules. For example, when lower emulsification speeds are used (*e.g.*, 1,000 to 2,000 rpm) are used, the droplets of the loading substance are large enough to form a single particle, which upon encapsulation, produces a single core microcapsule. Conversely, ifhigh emulsification speeds (*e.g.*, 5,000 to 15,000 rpm), the resultant droplets of loading substance are usually small (*e.g.*, from 1 to 10 µm). These tiny droplets can have higher surface energy and can readily form agglomerations when pH and/or temperature is adjusted accordingly, which results in the formation of multicore microcapsules upon encapsulation.

The emulsification step can be performed at greater than room temperature, greater than 3 0, 40, 50, 60, 70, or 80°C, where any of the stated values can form an upper or lower endpoint when appropriate. Specific examples include emulsifying the mixture at from about 30°C to about 60°C or from about 40°C to about 50°C.

It is further contemplated that antioxidants, which are also described herein, can be added to the aqueous mixture. Such antioxidants can be added before the emulsifying step, during the emulsifying step, and/or after the emulsifying step.

It is also contemplated that after the disclosed emulsions are prepared, the emulsions can be dehydrated. Methods for dehydrating emulsions are known in the art and include, but are not limited to, spray drying, freeze drying, evaporation, and the like.

Some specific processes for preparing emulsions disclosed herein involve the use of a first polymer component that is 240 Bloom fish gelatin, a loading substance that is microbial oil, and a pH from about 9 to about 11. Other examples include the use of a first polymer component that is 240 Bloom fish gelatin, a loading substance that is microbial oil, and a pH from about 3.5 to about 4.9. Further examples include the use of a first polymer component that is 0 Bloom fish gelatin, a loading substance that is microbial oil, and pH from about 9 to about 11. Still other examples include the use of a first polymer component that is 0 Bloom fish gelatin, a loading substance that is microbial oil, and a pH from about 3.5 to about 4.9.

It is also contemplated to additionally add a surfactant or a mixture of surfactants, which are also described herein. Such surfactants can be added before the emulsifying step, during the emulsifying step, and/or after the emulsifying step.

### Methods of Making Microcapsules

Methods for preparing the microcapsules disclosed herein are also described. In general, disclosed herein are processes for preparing microcapsules that comprise providing an emulsion comprising a first polymer component and a loading substance, wherein the emulsion has a pH of greater than about 6.0 or less than about 5.0; adding a second polymer component to the emulsion; adjusting pH, temperature, concentration, mixing speed, or a combination thereof to form an aqueous mixture comprising a primary shell material, wherein the primary shell material comprises the first and second polymer components and surrounds the loading substance; cooling the aqueous mixture to a temperature above the gel point of the primary shell material until the primary shell material forms agglomerations; and further cooling the aqueous mixture to form an outer shell around the agglomeration.

In the processes for preparing microcapsules disclosed herein, the emulsion can be provided according to the same methods disclosed herein for emulsion preparation. That is, any of the emulsions disclosed herein are suitable for use in the disclosed methods for preparing any of the microcapsules disclosed herein. Further, the emulsification temperature can initially be from about 30°C to about 60°C or from about 40°C to about 50°C. Also, emulsifying can be achieved by exposing a mixture of first polymer component and loading substance to high shear conditions (*e.g.*, from about 1,000 to about 15,000 rpm).

In the disclosed methods for preparing microcapsules, any of the first polymer components and loading substances disclosed herein for emulsions and microcapsules can be used. Also, the loading substance can be provided in an amount of from about 1% to about 50% by weight of the aqueous mixture.

Also, the emulsion pH and/or aqueous mixture pH can be greater than about 6 or less than about 5 (*e.g.,* from about 3.5 to about 4.9, from about 9.0 to about 11.0, or any of pH disclosed herein). An emulsion and/or aqueous mixture pH of greater than about 6.0 can be achieved by adding a basic substance as disclosed herein (*e.g.*, sodium hydroxide). Likewise, an emulsion and/or aqueous mixture pH of less than about 5.0 can be achieved by adding an acidic substance as disclosed herein (*e.g.*, phosphoric acid). Any of the pH ranges disclosed herein can be desirable for the disclosed microcapsule preparation methods. Determining the pH of the emulsions and/or aqueous mixtures in the methods for preparing microcapsules disclosed herein can be accomplished by methods known in the art (*e.g.*, pH meter, titration, etc.).

The use of emulsions with pH above about 6.0 and below about 5.0 as described herein can be a significant feature. Specifically, loading substances with higher interfacial tension typically produce stable emulsions that last through the complex coacervation process, resulting in encapsulated agglomerations. But when the loading substances have low interfacial tension, as is defined herein, the emulsions typically are not stable and will coalesce after shearing and during complex coacervation to produce single core, rather than multicore microparticles. Lowering or raising the pH of the emulsified low interfacial tension oil stabilized the emulsion and results in mulicore microcapsules.

The second polymer component that is used in the disclosed methods can be any of the materials disclosed herein for the first polymer component. For example, the second polymer component can comprise a surfactant, gelatin, polyphosphate, polysaccharide, or a mixture thereof. Other examples of the second polymer component include, but are not limited to, gelatin type A, gelatin type B, polyphosphate; gum arabic, alginate, chitosan, carrageenan, pectin, low methoxyl pectin, starch, modified starch, alpha-lactalbumin, beta-lactoglobumin, ovalbumin, polysorbiton, maltodextrin, cyclodextrin, cellulose, methyl cellulose, ethyl cellulose, hydropropylmethylcellulose, carboxymethylcellulose, milk protein, whey protein, soy protein, canola protein, albumin, xanthan, gellan gum, agar, kosher gelatin, non-kosher gelatin, Halal gelatin, and non-Halal gelatin, including mixtures and combinations thereof. In a particular example, the second the second polymer component can be polyphosphate.

After the second polymer is added the pH of the resulting mixture can be the same as that disclosed herein for the emulsions. That is, the pH can be greater than about 6.0 or less than about 5.0, including any and all ranges and points disclosed herein.

In the disclosed methods, the pH, temperature, concentration, mixing speed, or a combination thereof can be adjusted to form an aqueous mixture comprising a primary shell material, wherein the primary shell material comprises the first and second polymer components and surrounds the loading substance. The pH adjustment depends on the type of shell material to be formed. For example, the pH may be adjusted to a value from 3.5 to 5.0, or from 4.0 to 5.0. If the pH of the mixture starts in the desired range, then little or no pH adjustment is required. In one aspect, the initial temperature of the aqueous mixture is from about 20°C to about 60°C, or about 30°C to about 50°C. Mixing can be adjusted so that there is good mixing without breaking the microcapsules as they form. Particular mixing parameters depend on the type of equipment being used. Any of a variety of types of mixing equipment known in the art may be used. In one example, an axial flow impeller, such as Lightnin A310 or A510, can be used.

In many examples disclosed herein, the primary shell and the outer shell of the disclosed microcapsules can comprise a complex coacervate. The complex coacervate can be formed from the first and second polymer components. For example, the primary shell and the outer shell can comprise a complex coacervate between gelatin and polyphosphate. All combinations of first and second polymer components are contemplated herein for the complex coacervate and the primary and outer shell.

The aqueous mixture can then be cooled under controlled cooling rate and mixing parameters to permit agglomeration of the primary shells to form encapsulated agglomerations of primary shells. Not wishing to be bound by theory, the encapsulated agglomerations are discrete particles themselves. It is advantageous to control the formation of the encapsulated agglomerations at a temperature above the gel point of the shell material, and to let excess shell material form a thicker outer shell. It is also possible at this stage to add more polymer, where the polymer is the same or different, in order to thicken the outer shell and/or produce microcapsules having primary and outer shells of different composition. The outer shell encapsulates the agglomeration of primary shells to form a rigid encapsulated agglomeration of microcapsules.

Cooling the aqueous mixture can be accomplished by methods known in the art (*e.g.*, the use of a chiller). The rate of cooling can be about 1°C per about 1 to about 100 minutes. For example, the rate of cooling can be about 1°C per about 1, 5, 10, 15, 20, 25, 30, 35, 40, 45, 50, 55, 60, 65, 70, 75, 80, 85, 90, 95, or 100 minutes, where any of the stated values can form an upper or lower endpoint when appropriate. In specific examples the rate of cooling can be about 1°C/5 minutes. Cooling can take place until the mixture reaches a temperature of from about 5°C to about 10°C, *e.g.*, about 5°C.

Processing aids can be included in the shell material (*e.g.*, primary or outer shells). Processing aids can be used for a variety of reasons. For example, they may be used to promote agglomeration of the primary microcapsules, stabilize the emulsion system, improve the properties of the outer shells, control microcapsule size, and/or to act as an antioxidant. In one aspect, the processing aid can be an emulsifier, a fatty acid, a lipid, a wax, a microbial cell (*e.g.*, yeast cell lines), a clay, or an inorganic compound (*e.g.*, calcium carbonate). Not wishing to be bound by theory, these processing aids can improve the barrier properties of the microcapsules. In one aspect, one or more antioxidants can be added to the shell material. Antioxidant properties are useful both during the process (*e.g.*, during coacervation and/or spray drying) and in the microcapsules after they are formed (*i.e.*, to extend shelf-life, etc). Preferably a small number of processing aids that perform a large number of functions can be used. In one aspect, the antioxidant can be a phenolic compound, a plant extract, or a sulphur-containing amino acid. In one aspect, ascorbic acid (or a salt thereof such as sodium or potassium ascorbate) can be used to promote agglomeration of the primary microcapsules, to control microcapsule size and to act as an antioxidant. The antioxidant can be used in an amount of about 100 ppm to about 12,000 ppm, or from about 1,000 ppm to about 5,000 ppm. Other processing aids such as, for example, metal chelators, can be used as well. For example, ethylene diamine tetraacetic acid can be used to bind metal ions, which can reduce the catalytic oxidation of the loading substance.

In the disclosed microcapsules, the shell material can also be cross-linked. Thus, the disclosed methods can further involve the addition of a cross-linker. The cross-linker can be added to further increase the rigidity of the microcapsules by cross-linking the shell material in both the outer and primary shells and to make the shells insoluble in both aqueous and oily media. In one aspect, the cross-linker is added after the outer shell of the microcapsule is produced. Any suitable cross-linker can be used and the choice of cross-linker can vary depending upon the selection of the first and second polymer component. In one aspect, the cross-linkers can be enzymatic cross-linkers (e.g. transglutaminase), aldehydes (*e.g.* formaldehyde or gluteraldehyde), tannic acid, alum or a mixture thereof. In another aspect, the cross-linker can be a plant extract or a phenolic. It is also contemplated that one or more loading substances (*e.g.*, antioxidants) can be used with the cross-linker. When the microcapsules are to be used to deliver a biologically active substance to an organism, the cross-linkers are preferably non-toxic or of sufficiently low toxicity. The amount of cross-linker used depends on the components selected and can be adjusted to provide more or less structural rigidity as desired. In one aspect, the amount of cross-linker that can be used is in the amount of about 0.1 % to about 5.0%, about 0.5% to about 5.0%, about 1.0% to about 5.0%, about 2.0% to about 4.0%, or about 2.5%, by weight of the first polymer component. In general, one skilled in the art may routinely determine the desired amount in any given case by simple experimentation. The cross-linker can be added at any stage of the process, however it, can typically be added after the cooling step.

Further, the disclosed microcapsules can be washed with water and/or dried to provide a free-flowing powder. Thus, the disclosed methods can comprise a drying step for the microcapsules. Drying can be accomplished by a number of methods known in the art such as, for example, freeze drying, drying with ethanol, or spray drying. In one aspect, spray drying can be used for drying the microcapsules. Spray drying techniques are disclosed in "Spray Drying Handbook", K. Masters, 5th edition, Longman Scientific Technical UK, 1991, the disclosure of which is hereby incorporated by reference. In one example, the microcapsules can be co-spray dried with Zinc.

### Formulation Vehicles

Also disclosed herein are formulation vehicles comprising the microcapsules and/or emulsions disclosed herein. Any of the emulsions and/or microcapsules described herein can be incorporated into a formulation vehicle. Examples of formulation vehicles are provided herein and include, but are not limited to, foodstuffs, beverages, nutraceutical formulations, pharmaceutical formulations, lotions, creams, or sprays. In some other specific examples, the disclosed emulsions and/or microcapsules can be incorporated into gels, gel capsules, or tablets. Other vehicles include powders or powders coated with a polymer. Such vehicles can be given orally or, in the case of powders for example, sprinkled onto food or beverages.

### Supplements

Also, disclosed herein are nutritional supplements that comprise the emulsions and microcapsules disclosed herein. A nutritional supplement is any compound or composition that can be administered to or taken by a subject to provide, supply, or increase a nutrient(s) (*e.g.*, vitamin, mineral, essential trace element, amino acid, peptide, nucleic acid, oligonucleotide, lipid, cholesterol, steroid, carbohydrate, and the like). For example, a nutritional supplement can comprise a composition comprising one or more loading substances disclosed herein.

The nutritional supplement can comprise any amount of the emulsions and microcapsules disclosed herein, but will typically contain an amount determined to supply a subject with a desired dose of a loading substance (*e.g.*, EPA and/or DHA). The exact amount of emulsion or microcapsule required in the nutritional supplement will vary from subject to subject, depending on the species, age, weight and general condition of the subject, the severity of any dietary deficiency being treated, the particular mode of administration, and the like. Thus, it is not possible to specify an exact amount for every nutritional supplement. However, an appropriate amount can be determined by one of ordinary skill in the art using only routine experimentation given the teachings herein.

The nutritional supplement can also comprise other nutrient(s) such as vitamins other trace elements, minerals, and the like. Further, the nutritional supplement can comprise other components such as preservatives, antimicrobials, anti-oxidants, chelating agents, thickeners, flavorings, diluents, emulsifiers, dispersing aids, or binders.

The nutritional supplements are generally taken orally and can be in any form suitable for oral administration. For example, a nutritional supplement can typically be in a tablet, gel-cap, capsule, liquid, sachets, or syrup form.

The nutritional supplements can be designed for humans or animals, based on the recommended dietary intake for a given individual. Such considerations are generally based on various factors such as species, age, and sex as described above, which are known or can be determined by one of skill in the art. In one example, the disclosed supplements can be used as a component of feed for animals such as, but not limited to, livestock (*e.g.*, pigs, chickens, cows, goats, horses, and the like) and domestic pets (*e.g.*, cats, dogs, birds, and the like).

### Pharmaceutical Formulations

Also, pharmaceutical formulations comprising the disclosed emulsions and microcapsules are disclosed herein. A suitable pharmaceutical formulation can comprise any of the disclosed compositions with a pharmaceutically acceptable carrier. For example, a pharmaceutical formulation can comprise one or more of the disclosed emulsions and/or microcapsules and a pharmaceutically acceptable carrier. The disclosed pharmaceutical formulations can be used therapeutically or prophylactically.

By "pharmaceutically acceptable" is meant a material that is not biologically or otherwise undesirable, *i.e.*, the material may be administered to a subject without causing any undesirable biological effects or interacting in a deleterious manner with any of the other components of the pharmaceutical formulation in which it is contained. The carrier would naturally be selected to minimize any degradation of the active ingredient and to minimize any adverse side effects in the subject, as would be well known to one of skill in the art.

Pharmaceutical carriers are known to those skilled in the art. These most typically would be standard carriers for administration of drugs to humans, including solutions such as sterile water, saline, and buffered solutions at physiological pH. Suitable carriers and their formulations are described in Remington: The Science and Practice of Pharmacy, 21st ed., Lippincott Williams & Wilkins, Philidelphia, PA, 2005, which is incorporated by reference herein for its teachings of carriers and pharmaceutical formulations. Typically, an appropriate amount of a pharmaceutically-acceptable salt is used in the formulation to render the formulation isotonic. Examples of the pharmaceutically-acceptable carrier include, but are not limited to, saline, Ringer's solution and dextrose solution. The pH of the solution can be from about 5 to about 8 (*e.g.*, from about 7 to about 7.5). Further carriers include sustained release preparations such as semipermeable matrices of solid hydrophobic polymers containing the disclosed compounds, which matrices are in the form of shaped articles, *e.g.*, films, liposomes, microparticles, or microcapsules. It will be apparent to those persons skilled in the art that certain carriers can be more preferable depending upon, for instance, the route of administration and concentration of composition being administered. Other compounds can be administered according to standard procedures used by those skilled in the art.

Pharmaceutical formulations can include additional carriers, as well as thickeners, diluents, buffers, preservatives, surface active agents and the like in addition to the compounds disclosed herein. Pharmaceutical formulations can also include one or more additional active ingredients such as antimicrobial agents, anti-inflammatory agents, anesthetics, and the like.

The pharmaceutical formulation can be administered in a number of ways depending on whether local or systemic treatment is desired, and on the area to be treated. Administration can be topically (including ophthalmically, vaginally, rectally, intranasally), orally, by inhalation, or parenterally, for example by intravenous drip, subcutaneous, intraperitoneal or intramuscular injection. The disclosed compounds can be administered intravenously, intraperitoneally, intramuscularly, subcutaneously, intracavity, or transdermally.

Preparations for parenteral administration include sterile aqueous or non-aqueous solutions, suspensions, and emulsions. Examples of non-aqueous solvents are propylene glycol, polyethylene glycol, vegetable oils such as olive oil, marine oils, and injectable organic esters such as ethyl oleate. Aqueous carriers include water, alcoholic/aqueous solutions, and emulsions or suspensions, including saline and buffered media. Parenteral vehicles include sodium chloride solution, Ringer's dextrose, dextrose and sodium chloride, lactated Ringer's, and fixed oils. Intravenous vehicles include fluid and nutrient replenishers, electrolyte replenishers (such as those based on Ringer's dextrose), and the like. Preservatives and other additives may also be present such as, for example, antimicrobials, anti-oxidants, chelating agents, and inert gases and the like.

Pharmaceutical formulations for topical administration may include ointments, lotions, creams, gels, drops, suppositories, sprays, liquids and powders. Conventional pharmaceutical carriers, aqueous, powder or oily bases, thickeners and the like can be desirable.

Pharmaceutical formulations for oral administration include, but are not limited to, powders or granules, suspensions or solutions in water or non-aqueous media, capsules, sachets, or tablets. Thickeners, flavorings, diluents, emulsifiers, dispersing aids, or binders can be desirable.

Some of the formulations can potentially be administered as a pharmaceutically acceptable acid- or base-addition salt, formed by reaction with inorganic acids such as hydrochloric acid, hydrobromic acid, perchloric acid, nitric acid, thiocyanic acid, sulfuric acid, and phosphoric acid, and organic acids such as formic acid, acetic acid, propionic acid, glycolic acid, lactic acid, pyruvic acid, oxalic acid, malonic acid, succinic acid, maleic acid, and fumaric acid, or by reaction with an inorganic base such as sodium hydroxide, ammonium hydroxide, potassium hydroxide, and organic bases such as mono-, di-, trialkyl and aryl amines and substituted ethanolamines.

### Foodstuffs

Also disclosed herein are foodstuffs that comprise any of the disclosed emulsions and microcapsules. By "foodstuff" is.meant any article that can be consumed (*e.g.*, eaten, drank, or ingested) by a subject. In one example, the disclosed compositions can be used as nutritional supplements that are added to a foodstuff. For example, the disclosed emulsions and/or microcapsules can be added to food or beverages. In this sense, the disclosed compositions can be prepared in, for example, a powdered form and contained in articles such as sachets or shakers, which can be used to pour or sprinkle the disclosed compositions onto and into food and beverages.

In some examples, the foodstuff is a baked good, a pasta, a meat product, a frozen dairy product, a milk product, a cheese product, an egg product, a condiment, a soup mix, a snack food, a nut product, a plant protein product, a hard candy, a soft candy, a poultry product, a processed fruit juice, a granulated sugar (*e.g.*, white or brown), a sauce, a gravy, a syrup, a nutritional bar, a beverage, a dry beverage powder, a jam or jelly, a fish product, or pet companion food. In other examples, the foodstuff is bread, tortillas, cereal, sausage, chicken, ice cream, yogurt, milk, salad dressing, rice bran, fruit juice, a dry beverage powder, liquid beverage, rolls, cookies, crackers, fruit pies, or cakes.

### Methods of Use

The disclosed emulsions and/or microcapsules also have a wide variety of uses. For example, disclosed herein are methods of delivering a loading substance to a subject by administering to the subject a microcapsule and/or an emulsion as disclosed herein. Also disclosed is the use a microcapsule and/or emulsion as disclosed herein to prepare a medicament for delivering a loading substance to a subject.

In a particular example, the disclosed emulsions and/or microcapsules (including nutritional supplements, pharmaceutical formulations, delivery devices, and foodstuffs that contains the disclosed emulsions and/or microcapsules) can be used as a source of fatty acids (*e.g.*, omega-3 fatty acids), lowering triglycerides and influencing diabetes related biochemistry. In another particular example, disclosed herein are methods of supplementing omega-3 fatty acids in a subject by administering an effective amount of an emulsion and/or microcapsule disclosed herein, wherein the loading substance comprises an omega-3 fatty acid. In another example, disclosed herein are methods of lowering cholesterol levels, triglyceride levels, or a combination thereof in a subject by administering an effective amount of an emulsion and/or microcapsule disclosed herein.

Omega-3 fatty acids are vital to everyday life and function. For example, the beneficial effects of omega-3 fatty acids like *cis-*5,8,11,14,17*-*eicosapentaenoic acid (EPA) and *cis*-4,7,10,13,16,19-docosahexaenoic acid (DHA) on lowering serum triglycerides are well established. These compounds are also known for other cardioprotective benefits such as preventing cardiac arrhythmias, stabilizing atherosclerotic plaques, reducing platelet aggregation, and reducing blood pressure. *See e.g.*, Dyrberg et al., In: Omega-3 Fatty Acids: Prevention and Treatment of Vascular Disease. Kristensen et al., eds., Bi & Gi Publ., Verona-Springer-Verlag, London, pp. 217-26, 1995; O'Keefe and Harris, Am. J. Cardiology 2000, 85:1239-41; Radack et al., "The effects of low doses of omega-3 fatty acid supplementation on blood pressure in hypertensive subjects: a randomized controlled trial." Arch. Intern. Med. 1991, 151:1173-80; Harris, "Extending the cardiovascular benefits of omega-3 fatty acids." Curr Atheroscler Rep 2005, 7:375-80; Holub, "Clinical nutrition: 4 omega-3 fatty acids in cardiovascular care." CMAJ 2002, 166(5):608-15. Indeed, the American Heart Association has also reported that omega-3 fatty acids can reduce cardiovascular and heart disease risk. Other benefits of omega-3 fatty acids are those related to the prevention and/or treatment of inflammation and neurodegenerative diseases, and to improved cognitive development. *See e.g.*, Sugano and Michihiro, "Balanced intake of polyunsaturated fatty acids for health benefits." J. Oleo Sci. 2001, 50(5):305-11.

The fatty acids EPA and DHA can be synthesized in the human body from α-linolenic acid (18:3); however, the conversion rate from this precursor molecule is limited (Muskiet et al., "Is docosahexaenoic acid (DHA) essential? Lessons from DHA status regulation, our ancient diet, epidemiology and randomized controlled trials." J. Nutr. 2004, 134(1):183-6). Accordingly, EPA and DHA in the body are primarily derived from dietary sources (*e.g.*, oily fish). Diets rich in fish oils are known to have many beneficial effects for heart disease, cancer, arthritis, allergies, and other chronic diseases. Epidemiological clinical trials have shown that increasing the dietary intake of omega-3 fatty acids, in the form of fish or of fish oil supplements, may reduce various risk factors associated with cardiovascular disease. *See e.g.*, The American Heart Association, Scientific Statement, "Fish Consumption, Fish Oil, Omega-3 Fatty Acids and Cardiovascular Disease," November 2002; Appel et al., "Does supplementation of diet with 'fish oil' reduce blood pressure? A meta-analysis of controlled clinical trials." Arch. Intern. Med. 1993, 153(12):1429-1438; GISSI-Prevenzione Investigators. "Dietary supplementation with omega-3 polyunsaturated fatty acids and vitamin E after myocardial infarction: results of the GISSI-Prevenzione trial." Lancet 1999, 354:447-55.

Despite the strong evidence for the benefit of omega-3 fatty acids like EPA and DHA in prevention of cardiovascular disease, the average daily consumption of these fatty acids by North Americans is estimated to be between 0.1 to 0.2 grams, compared to a suggested daily intake of 0.65 grams to confer benefit (Webb, "Alternative sources of omega-3 fatty acids." Natural Foods Merchandiser 2005, XXVI(8):40-4). Since altering dietary patterns of populations is difficult and many people do not like to eat fish, dietary supplementation with EPA and DHA is an important approach to addressing this problem. Unfortunately, many supplements of omega-3 fatty acids are sensitive to oxidation and can be foul smelling and tasting. Further, compliance with dietary supplement regimens requires discipline, which is often wanting. In light of the health benefits of omega-3 fatty acids, the disclosed emulsions and/or microcapsules can be used to deliver omega-3 fatty acids to a subject.

In the disclosed methods of use, the emulsions and/or microcapsules that are administered can be any of the compositions disclosed herein. For example, the disclosed emulsions and/or microcapsules can be used in the disclosed methods in the form of any of the nutritional supplements disclosed herein. In another example, the disclosed emulsions and/or microcapsules can be used in the disclosed methods in the form of any of the pharmaceutical formulations disclosed herein. In still another example, the disclosed emulsions and/or microcapsules can be incorporated in any of the delivery devices disclosed herein, or incorporated into any foodstuff disclosed herein and used in the disclosed methods.

It is contemplated that the methods disclosed herein can be accomplished by administering various forms of the disclosed emulsions and/or microcapsules. For example, one can administer any of the pharmaceutical formulations with any of the foodstuffs disclosed herein. In another example, one can administer a tablet or capsule with any of the nutritional supplements disclosed herein. In yet another example, one can administer any of the pharmaceutical formulations with any of the delivery devices and nutritional supplement disclosed herein, and the like.

### Dosage

When used in the above described methods or other treatments, or in the nutritional supplements, pharmaceutical formulations, delivery devices, or foodstuffs disclosed herein, an "effective amount" of one of the disclosed emulsions and/or microcapsules can be employed in pure form or, where such forms exist, in pharmaceutically acceptable salt form, and with or without a pharmaceutically acceptable excipient, carrier, or other additive.

The specific effective dose level for any particular subject will depend upon a variety of factors including the disorder being treated and the severity of the disorder; the identity and activity of the specific composition employed; the age, body weight, general health, sex and diet of the patient; the time of administration; the route of administration; the rate of excretion of the specific composition employed; the duration of the treatment; drugs used in combination or coincidental with the specific composition employed and like factors well known in the medical arts. For example, it is well within the skill of the art to start doses of a composition at levels lower than those required to achieve the desired therapeutic effect and to gradually increase the dosage until the desired effect is achieved. If desired, the effective daily dose can be divided into multiple doses for purposes of administration. Consequently, single dose compositions can contain such amounts or submultiples thereof to make up the daily dose.

The dosage can be adjusted by the individual physician or the subject in the event of any counterindications. Dosage can vary, and can be administered in one or more dose administrations daily, for one or several days. Guidance can be found in the literature for appropriate dosages for given classes of pharmaceutical products.

Further, disclosed are methods for delivering a disclosed composition to a subject by administering to the subject any of the nutritional supplements, pharmaceutical formulations, delivery devices, and/or foodstuffs disclosed herein. The disclosed compositions (including nutritional supplements, delivery devices, and Pharmaceutical formulations) can typically be administered orally.

### EXAMPLES

The following examples are set forth below to illustrate the methods and results according to the disclosed subject matter. These examples are not intended to be inclusive of all aspects of the subject matter disclosed herein, but rather to illustrate representative methods and results. These examples are not intended to exclude equivalents and variations of the present invention which are apparent to one skilled in the art.

Efforts have been made to ensure accuracy with respect to numbers (*e.g.*, amounts, temperature, pH, etc.) but some errors and deviations should be accounted for. Unless indicated otherwise, parts are parts by weight, temperature is in °C or is at ambient temperature, and pressure is at or near atmospheric. There are numerous variations and combinations of conditions, *e.g.*, component concentrations, temperatures, pressures, and other reaction ranges and conditions that can be used to optimize the product purity and yield obtained from the described process. Only reasonable and routine experimentation will be required to optimize such process conditions.

Certain materials, compounds, compositions, and components disclosed herein can be obtained commercially or readily synthesized using techniques generally known to those of skill in the art. For example, the starting materials and reagents used in preparing the disclosed compositions are either available from commercial suppliers such as Ocean Nutrition Canada, Ltd. (Dartmouth, Canada), Martek Biosciences Corp. (Columbia, MD) Aldrich Chemical Co., (Milwaukee, Wis.), Acros Organics (Morris Plains, N.J.), Fisher Scientific (Pittsburgh, Pa.), or Sigma (St. Louis, Mo.) or are prepared by methods known to those skilled in the art following procedures set forth in references such as Fieser and Fieser's Reagents for Organic Synthesis, Volumes 1-17 (John Wiley and Sons, 1991); Rodd's Chemistry of Carbon Compounds, Volumes. 1-5 and Supplementals (Elsevier Science Publishers, 1989); Organic Reactions, Volumes 1-40 (John Wiley and Sons, 1991); March's Advanced Organic Chemistry, (John Wiley and Sons, 4th Edition); and Larock's Comprehensive Organic Transformations (VCH Publishers Inc., 1989).

In the following examples the loading substance was either algal oil (DHASCO-S), which is commercially available from Martek Biosciences Corp., Columbian, MD, or high DHA fish oil (XODHA), which is commercially available from Ocean Nutrition Canada Ltd., Dartmouth, Canada. The algal oil DHASCO-S had an interfacial tension of 0.5 dynes/cm when measured against 3% gelatin at natural pH 6.35. The algal oil had an interfacial tension of 1.0 dynes/cm when measured against 3% gelatin at pH 4.02, an interfacial tension of 0.6 dynes/cm when measured against 3% gelatin at pH 8.34, and an interfacial tension of 0.7 dynes/cm when measured against 3% gelatin at pH 11.06.

### Example 1: Microalgal DHA oil microencapsulated in 240 Bloom fish gelatin (oil droplets stabilized by high pH)

240 Bloom fish gelatin (44.0 g) was dissolved in water (320 g) and the solution was heated to 40°C. Sodium ascorbate (1.6 g) was added to the gelatin solution and the solution pH was adjusted to 10 with a 10% NaOH solution. Algal oil (DHASCO-S, Martek Biosciences Corp.; 72.0 g) was then added to the gelatin solution and emulsified at 7500 rpm for 4 minutes. The emulsion was examined under a microscope after emulsification and after sitting for 30 minutes to ensure the absence of oil droplet coalescence.

To a 2 liter reactor was added distilled water (1051 g) along with sodium ascorbate (5.7 g). The temperature of this solution was maintained at 40°C. The emulsion was then added to the distilled water in the reactor and the pH of the mixture was maintained at 10 by adding 10% NaOH solution. This diluted emulsion was mixed at 40°C for 2 hr.

Sodium polyphosphate (4.4 g) was dissolved in distilled water (84 g) and the solution was also added to the diluted emulsion in the reactor. The mixture in the reactor had a pH of 9.86. The pH was then adjusted to about 4.35 with 10% phosphoric acid to form about 30 µm agglomerations of the primary microcapsules.

Next, the mixture was cooled from 45°C to 5°C at an average cooling rate of 5°C/minute. After adjusting the pH to 5,1% w/w of a transglutaminase preparation was added. The slurry was held at 15°C for 7 hr for crosslinking, followed by enzymatic hardening at 20°C for 8 hr.

The finished suspension of microcapsules was then ready for applications in foods. It was also spray dried to produce a free flowing powder with surface free oil content below 0.1% w/w.

### Example 2: Microalgal DHA oil microencapsulated in 240 Bloom fish gelatin (oil droplets stabilized by low pH)

240 Bloom fish gelatin (44.0 g) was dissolved in water (320 g) and heated to 44°C. Sodium ascorbate (1.6 g) was added to the gelatin solution and the solution pH was adjusted to 4.65 with 10% phosphoric acid solution. Algal oil (DHASCO-S, Martek Biosciences Corp; 72.0 g) was added to the gelatin solution and then emulsified at 7500 rpm for 4 minutes. The emulsion was examined under a microscope after emulsification and after sitting for 30 minutes to confirm the absence of oil droplet coalescence.

To a 2 liter reactor was added distilled water (1051 g) along with sodium ascorbate (5.7 g). The temperature of this solution was maintained at 40°C. The emulsion was then added to the distilled water in the reactor.

Sodium polyphosphate (4.4 g) was dissolved in distilled water (84 g) and was also added to the diluted emulsion in the reactor. The mixture in the reactor had a pH of 4.69. The pH was then adjusted to 4.54 with 10% phosphoric acid to form about 30 µm agglomerations of the primary microcapsules.

Next, the mixture was cooled from 40°C to 5°C at an average cooling rate of 5°C/min. After adjusting the pH to 5,1% w/w of a transglutaminase preparation was added. The slurry was then held at 15°C for 7 hr for crosslinking, followed by enzymatic hardening at 20°C for 8 hr.

The finished suspension of microcapsules was then ready for applications in foods. It was also spray dried to produce a free flowing powder with surface free oil content below 0.1% w/w.

### Example 3: Microalgal DHA oil microencapsulated in 0 Bloom fish gelatin (oil droplets stabilized by tow pH)

Zero Bloom fish gelatin (44.0 g) was dissolved in water (320 g) and the solution was heated to 35°C. Sodium ascorbate (1.6 g) was added to the gelatin solution and the solution pH was adjusted to 4.15 with 10% phosphoric acid. Algal oil (DHASCO-S, Martek Biosciences Corp; 72.0 g) was added to the gelatin solution and emulsified for 4 minutes at 7500 rpm. The emulsion was monitored under a microscope after emulsification and after sitting for 30 minutes to ensure the absence of oil droplet coalescence.

To a 2 liter reactor was added distilled water (1051 g) along with sodium ascorbate (5.7 g). After stirring on a hot plate for 30 minutes at 35°C, the emulsion was added to the distilled water in the reactor.

Sodium polyphosphate (3.16 g) was dissolved in distilled water (80 g) and also added to the diluted emulsion in the reactor. The mixture in the reactor had a pH of 4.76. The pH was then adjusted to 4.69 with 10% phosphoric acid to give about 30 µm agglomerations of the primary microcapsules.

Next, the mixture was cooled from 35°C to 5°C at an average cooling rate of 5°C/mm. After adjusting the pH to 5, 1% w/w of a transglutaminase preparation was added. The slurry was then held at 5°C for 5 hr for crosslinking, followed by enzymatic hardening at 20°C for 9 hr.

The finished suspension of microcapsules was then ready for applications in foods. It was also spray dried to produce a free flowing powder with a surface free oil below 0.1 % w/w.

### Example 4: Microalgal DHA oil microencapsulated in 0 Bloom_fish gelatin (oil droplets stabilized by high pH)

Zero Bloom fish gelatin (44.0g) was dissolved in water (320 g) and the solution was heated to 35°C. Solution pH was adjusted to 10 with 10% NaOH solution. Algal oil (DHASCO-S; Martek Biosciences Corp; 72.0 g) was added to the gelatin solution and emulsified at 7500 rpm for 4 minutes.

To a 2 liter reactor was added distilled water (1051 g) along with sodium ascorbate (5.7 g). The temperature of this solution was maintained at 35°C and pH was adjusted to 10 (exact pH was 10.161). The emulsion was then added to the reactor and stirred at 35°C.

The diluted emulsion was examined under a microscope after emulsification and after sitting for 30 minutes to ensure the absence of oil droplet coalescence. This emulsion was then stirred and held at 35°C for another 1.5 hr (total = 2 hr) and was found to be stable in oil droplet size.

Sodium polyphosphate (4.4 g) was dissolved in distilled water (84 g) and then the solution was also added to the diluted emulsion in the reactor. The mixture in the reactor had a pH of 9.678. The pH was then adjusted to 4.473 with 10% phosphoric acid to form about 30 µm agglomerations of the primary microcapsules.

Next, the mixture was cooled from 35°C to 5°C at an average cooling rate of 5°C/min. After adjusting pH to 5, 1% w/w of a transglutaminase preparation was added. The slurry was then held at 5°C for 5 hr for crosslinking, followed by enzymatic hardening at 20°C for 9 hr.

The finished suspension of microcapsules was then ready for applications in foods. It was also spray dried to produce a free flowing powder with a surface free oil below 0.1 % w/w.

### Example 5 (control): Microalgal DHA oil microencapsulated in 240 Bloom fish gelatin

240 Bloom fish gelatin (44 g) was dissolved in water (320 g) and the solution was heated to 40°C. Sodium ascorbate (7.3 g) was added to the gelatin solution and the solution pH was 5.833. Algal oil (DHASCO-S, Martek Biosciences Corp; 72.0 g) was added to the gelatin solution and then emulsified at 7500 rpm for 4 minutes. The emulsion was examined under a microscope after emulsification and verified that the oil droplets were small and uniform (1-5 µm in diameter).

To a 2 liter reactor was added distilled water (1051 g) and the temperature was maintained at 40°C. The emulsion was added to distilled water in the reactor and the pH of the mixture was found be 5.749.

Sodium polyphosphate (4.4 g) was dissolved in distilled water (84 g) and the solution was also added to the diluted emulsion in the reactor. The mixture in the reactor had a pH of 6.541. Oil droplets were 1-5 µm and appeared similar to a regular fish oil emulsion.

The pH was then lowered with 10% phosphoric acid in order to form agglomerations of the primary microcapsules. A normal fish oil microencapsulation process would need to be done around pH 4.5 to 5.0. However, after pH was lowered to 4.840, the oil droplets become larger (50-150 µm in diameter) and free oil droplets were seen floating on the top of the slurry.

When the pH was further lowered to 4.784, oil droplets were larger, and more free oil droplets were seen on the surface of slurry. After the pH was adjusted to 4.601, the large coalescence oil droplets (50-154 µm) formed large clumps and no regular multicore microcapsules could be found in the slurry sample. The process then had to be terminated.

### Example 6 (control): DHA fish oil microencapsulated in 275 Bloom porkskin gelatin

275 Bloom porkskin gelatin (44 g) was dissolved in water (482 g) and the solution was heated to 50°C. The pH was 4.638. Sodium ascorbate (7.3 g) was added to the gelatin solution and the solution pH was 5.271. High DHA fish oil (XODHA; Ocean Nutrition Canada Ltd.; 72.0 g) was added to the gelatin solution and then emulsified at 7500 rpm for 4 minutes. The emulsion was examined under a microscope after emulsification and verified that the oil droplets were small and uniform (1-5 µm in diameter).

To a 2 liter reactor was added distilled water (890 g) and the temperature was maintained at 50°C. The emulsion was added to distilled water in the reactor and the pH of the mixture was found be 5.058.

Sodium polyphosphate (4.4 g) was dissolved in distilled water (84 g) and the solution was also added to the diluted emulsion in the reactor. The mixture in the reactor had a pH of 5.821 at 49.9°C. The pH was then lowered with 10% phosphoric acid in order to form agglomerations of the primary microcapsules. When pH was further lowered to 4.686, secondary microcapsules formed 30-50 µm in diameter.

Next, the mixture was cooled from 50°C to 4°C at an average cooling rate of 1°C/5 minutes. After adjusting pH to 6.0 by adding 10% NaOH, 1 % w/w of a transglutaminase preparation (Ajinomoto USA Inc., Fort Lee, NJ) was added. The slurry was then held at room temperature (25°C) for 16 hr for crosslinking.

The slurry was ready for food applications. It was spray dried to produce a free flowing powder, which had an induction period of 44.7 hr determined at 65°C under initial pressure of approximately 550 kPa of oxygen by using Oxipres (Mikrolab Aarhus A/S, Hojbjerg, DNK).

### Example 7 (control): DHA fish oil microencapsulated in 240 Bloom fish gelatin

240 Bloom fish gelatin (44 g) was dissolved in water (320 g) and the solution was heated to 40°C. The pH was 5.807. Sodium ascorbate (7.3 g) was added to the gelatin solution and the solution pH was 5.942. High DHA fish oil (XODHA; Ocean Nutrition Canada Ltd.; 72.0 g) was added to the gelatin solution and then emulsified at 7500 rpm for 4 minutes. The emulsion was examined under a microscope after emulsification and verified that the oil droplets were small and uniform (1-5 µm in diameter).

To a 2 liter reactor was added distilled water (1051 g) and the temperature was maintained at 40°C. The emulsion was added to distilled water in the reactor and the pH of the mixture was found be 5.812.

Sodium polyphosphate (4.4 g) was dissolved in distilled water (84 g) and the solution was also added to the diluted emulsion in the reactor. The mixture in the reactor had a pH of 6.512.

The pH was then lowered with 10% phosphoric acid in order to form agglomerations of the primary microcapsules. When the pH was further lowered to 4.773, secondary microcapsules formed 30-50 µm agglomerations.

Next, the slurry was cooled from 40°C to 5°C at an average cooling rate of 1°C/5 minutes. After adjusting pH to 6.0 by adding 10% NaOH, 1% w/w of a transglutaminase preparation (Ajinomoto USA Inc., Fort Lee, NJ) was added for crosslinking. The shells of the microcapsules were hardened at 5°C for 1 hr, 15°C for 8 hr, and 20°C for 9 hr. The slurry was then ready for food applications. It was spray dried to produce a free flowing powder. It had an induction period of 43.5 hrs.

### Example 8 (control): DHA fish oil microencapsulated in 0 Bloom fish gelatin

Zero Bloom fish gelatin (44 g) was dissolved in water (323 g) and the solution was heated to 35.6°C. Sodium ascorbate (7.3 g) was added to the gelatin solution and the solution pH was 6.042. Sodium polyphosphate (4.4 g) was dissolved in distilled water (84 g) and the solution was added to the gelatin solution. The mixture had a pH of 6.306 at 34.1°C. The pH was then adjusted to 4.9 with 10% phosphoric acid.

High DHA fish oil (XODHA; Ocean Nutrition Canada Ltd.; 72.6 g) was added to the gelatin solution and then emulsified at 7500 rpm for 4 minutes. The emulsion was examined under a microscope after emulsification and verified that the oil droplets were small and uniform (1-5 µm in diameter).

To a 2 liter reactor was added distilled water (1060 g) and the temperature was maintained at 35°C. The emulsion was added to distilled water in the reactor and the pH of the mixture was found be 4.941.

While the mixture was agitated, the pH was then lowered with 10% phosphoric acid in order to form agglomerations of the primary microcapsules. When the pH was further lowered to 4.751, secondary microcapsules formed about 40 µm agglomerations.

Next, the mixture was cooled from 35°C to 5°C at an average cooling rate of 1°C/5 minutes. After adjusting pH to 6.0 by adding 10% NaOH, 1% w/w of a transglutaminase preparation was added. The slurry was then held at 5°C for 5 hr for crosslinking, followed by enzymatic hardening at 20°C for 10 hr. The suspension was ready for food applications. It was also spray dried to produce a free flowing powder. It had an induction period of 36.9 hrs.

### Example 9: Microencapsulation using SPI/agar/gellan gum with algal oil

26.67 g of soy protein isolates (ICN Biomedicals, Inc.) was dissolved in 220.0 g of distilled water. The resulting solution was heated up to 60°C and the pH was adjusted to 10.6.

40.0 g of algal oil was heated to 50°C. The algal oil was then added to the soy protein solution and emulsified at 8000 rpm for 5 minutes. The emulsion was examined under a microscope after emulsification to verify that the oil droplets were about 1 µm in diameter.

2.0 g of agar (TIC pretested agar, TIC Gums) was dissolved in 66.7.0 of boiling distilled water and then transferred to a 2-L reactor with 400.0 g of distilled water and 3.33 g of sodium ascorbate. The temperature in the reactor was maintained at 55°C and the mixture had a pH of about 7.0.

The algal oil emulsion was added to the distilled water in the reactor and the pH of the mixture was about 10.2. The pH was then adjusted to about 5.7 with 10% w/w phosphoric acid to form about 30 µm agglomerations of the primary microcapsules.

2.1 g of transglutaminase in 10.0 g of distilled water was next added to the reactor and the mixture was maintained at 50°C for 3 hours before cooling down to 44°C.

2.67 g of gellan gum (Kelcogel F) and 1.33 g of sodium ascorbate were dissolved in 266.7 g of distilled water at 65°C and then cooled to 50°C. 2.6 g of SPI was dissolved in 30.0 g of distilled water with pH adjusted to about 9. The SPI solution was then mixed with the gellan gum solution and pH was adjusted to about 6.7. The resulting SPI/gellan gum solution was then added to the agglomerated primary microcapsules in the reactor at 44°C.

1.0 g of CaCl₂ in 5.0g distilled water was added to the reactor and the agitation speed was gradually increased as the solution was quickly cooled down to 20°C. The finished suspension of microcapsules had a compact structure and shell, and the shell survived after boiling. Such a microcapsule would be suitable for a vegan, lactovegetarian, ovo-lactovegetarian, and semi-vegetarian diet.

It will be apparent to those skilled in the art that various modifications and variations can be made in the present invention without departing from the scope or spirit of the invention. Other embodiments of the invention will be apparent to those skilled in the art from consideration of the specification and practice of the invention disclosed herein. It is intended that the specification and examples be considered as exemplary only, with a true scope and spirit of the invention being indicated by the following claims.

The following are embodiments of the invention:
A. An emulsion, comprising a first polymer component and a loading substance, wherein the loading substance comprises microbial oil and has an interfacial tension of less than about 5 dynes/cm and wherein the emulsion has a pH of greater than about 6.0 or less than about 5.0.
B. The emulsion according to embodiment A, further comprising an antioxidant.
C. The emulsion according to any of the preceding embodiments, wherein the antioxidant comprises a phenolic compound, a plant extract, or a sulphur-containing compound.
D. The emulsion according to any of the preceding embodiments, wherein the antioxidant comprises ascorbic acid or a salt thereof.
E. The emulsion according to any of the preceding embodiments, further comprising sodium hydroxide, phosphoric acid, or a mixture thereof.
F. The emulsion according to any of the preceding embodiments, wherein the pH is from about 3.5 to about 4.9.
G. The emulsion according to any of the preceding embodiments, wherein the pH is from about 9.0 to about 11.0.
H. The emulsion according to any of the preceding embodiments, wherein the first polymer component comprises a surfactant, gelatin, polyphosphate, polysaccharide, or a mixture thereof.
1. The emulsion according to any of the preceding embodiments, wherein the first polymer - component comprises gelatin type B, polyphosphate, gum arabic, alginate, chitosan, carrageenan, pectin, low methoxyl pectin, starch, modified starch, alpha-lactalbumin, beta-lactoglobumin, ovalbumin, polysorbiton, maltodextrin, cyclodextrin, cellulose, methyl cellulose, ethyl cellulose, hydropropylmethylcellulose, carboxymethylcellulose, milk protein, whey protein, soy protein, canola protein, albumin, xanthan, gellan gum, agar, kosher gelatin, non-kosher gelatin, Halal gelatin, non-Halal gelatin, or a mixture thereof.
J. The emulsion according to any of the preceding embodiments, wherein the first polymer component comprises gelatin type A.
K. The emulsion according to any of the preceding embodiments, wherein the first polymer component comprises fish gelatin.
L. The emulsion according to any of the preceding embodiments, wherein the first polymer component has a Bloom number of from about 0 to about 300.
M. The emulsion according to any of the preceding embodiments, wherein the first polymer component has a Bloom number of from about 0 to about 50.
N. The emulsion according to any of the preceding embodiments, wherein the first polymer component has a Bloom number of from about 51 to about 300.
O. The emulsion according to any of the preceding embodiments, wherein the first polymer component has a Bloom number of about 0, about 210, about 220, or about 240.
P. The emulsion according to any of the preceding embodiments, wherein the loading substance has an interfacial tension of less than about 2 dynes/cm.
Q. The emulsion according to any of the preceding embodiments, wherein the loading substance has an interfacial tension of less than about 1 dynes/cm.
R. The emulsion according to any of the preceding embodiments, wherein the loading substance has an interfacial tension of less than about 0.5 dynes/cm.
S. The emulsion according to any of the preceding embodiments, wherein the loading substance comprises an algal oil.
T. The emulsion according to any of the preceding embodiments, wherein the loading substance comprises oil from a dinoflagellate.
U. The emulsion according to any of the preceding embodiments, wherein the loading substance comprises oil from Crypthecodiniwn cohnii.
V. The emulsion according to any of the preceding embodiments, wherein the loading substance comprises fungal oil.
W. The emulsion according to any of the preceding embodiments, wherein the loading substance comprises oil from Thraustochytrium, Schizochytrium, or a mixture thereof.
X. The emulsion according to any of the preceding embodiments, wherein the loading substance comprises an omega-3 fatty acid, an alkyl ester of an omega-3 fatty acid, a triglyceride ester of an omega-3 fatty acid, a phytosterol ester of an omega-3 fatty acid, and/or a mixture thereof.
Y. The emulsion according to any of the preceding embodiments, wherein the loading substance comprises docosahexaenoic acid and/or eicosapentaenoic acid, a C₁-C₆ alkyl ester thereof, a triglyceride ester thereof, a phytosterol ester thereof, and/or a mixture thereof.
Z. The emulsion according to any of the preceding embodiments, wherein the loading substance is from about 1% to about 50% by weight of the emulsion.
AA. The emulsion according to any of the preceding embodiments, wherein the first polymer component is 240 Bloom fish gelatin, he loading substance is algal oil, and the pH is from about 9 to about 11.
AB. The emulsion according to any of the preceding embodiments, wherein the first polymer component is 240 Bloom fish gelatin and the pH is from about 3.5 to about 4.9.
AC. The emulsion according to any of the preceding embodiments, wherein the first polymer component is 0 Bloom fish gelatin and the pH is from about 9 to about 11.
AD. The emulsion according to any of the preceding embodiments, wherein the first polymer component is 0 Bloom fish gelatin and the pH is from about 3.5 to about 4.9.
AE. The emulsion according to any of the preceding embodiments, wherein the emulsion contains an average droplet size of less than about 1000 nm.
AF. The emulsion according to any of the preceding embodiments, wherein the emulsion contains an average droplet size of less than about 500 nm
AG. The emulsion according to any of the preceding embodiments, wherein the emulsion contains an average droplet size of less than about 100 nm.
AH. The emulsion according to any of the preceding embodiments, further comprising a surfactant.
AI. A process for preparing an emulsion, comprising providing an aqueous mixture of a first polymer component and a loading substance, wherein the loading substance comprises a marine oil and has an interfacial tension of less than about 5 dynes/cm and wherein the mixture has a pH of greater than about 6.0 or less than about 5.0; and emulsifying the mixture.
AJ. The process according to embodiment AI, wherein an antioxidant is added to the aqueous mixture.
AK. The process according to any of embodiments AI-AJ, wherein the antioxidant comprises a phenolic compound, a plant extract, or a sulphur-containing compound.
AL. The process according to any of embodiments AI-AK, wherein the antioxidant comprises ascorbic acid or a salt thereof.
AM. The process according to any of embodiments AI-AL, wherein the pH of greater than about 6.0 is achieved by adding sodium hydroxide or wherein the pH of less than about 5.0 is achieved by adding phosphoric acid.
AN. The process according to any of embodiments AI-AM, wherein the pH is from about 3.5 to about 4.9.
AO. The process according to any of embodiments AI-AN, wherein the pH is from about 9.0 to about 11.0.
AP. The process according to any of embodiments AI-AO, wherein emulsifying is performed at a temperature of from about 30°C to about 60°C.
AQ. The process according to any of embodiments AI-AP, wherein emulsifying is performed at a temperature of from about 40°C to about 50°C.
AR. The process according to any of embodiments AI-AQ, wherein the mixture is emulsified at from about 1,000 to about 15,000 rpm.
AS. The process according to any of embodiments AI-AR, further comprising dehydrating the emulsion.
AT. The process according to any of embodiments AI-AS, wherein the first polymer component comprises a surfactant, gelatin, polyphosphate, polysaccharide, or a mixture thereof.
AU. The process according to any of embodiments AI-AT, wherein the first polymer component comprises gelatin type B, polyphosphate, gum arabic, alginate, chitosan, carrageenan, pectin, low methoxyl pectin, starch, modified starch, alpha- lactalbumin, beta-lactoglobumin, ovalbumin, polysorbiton, maltodextrin, cyclodextrin, cellulose, methyl cellulose, ethyl cellulose, hydropropylmethylcellulose, carboxymethylcellulose, milk protein, whey protein, soy protein, canola protein, albumin, xanthan, gellan gum, agar, kosher gelatin, non-kosher gelatin, Halal gelatin, non-Halal gelatin, or a mixture thereof.
AV. The process according to any of embodiments AI-AU, wherein the first polymer component comprises gelatin type A.
AW. The process according to any of embodiments AI-AV, wherein the first polymer component comprises fish gelatin.
AX. The process according to any of embodiments AI-AW, wherein the first polymer component has a Bloom number of from about 0 to about 300.
AY. The process according to any of embodiments AI-AX, wherein the first polymer component has a Bloom number of from about 0 to about 50.
AZ. The process according to any of embodiments AI-AY, wherein the first polymer component has a Bloom number of from, about 51 to about 300.
BA. The process according to any of embodiments AI-AZ, wherein the first polymer component has a Bloom number of about 0, about 210, about 220, or about 240.
BB. The process according to any of embodiments AI-BA, wherein the loading substance has an interfacial tension of less than about 2 dynes/cm.
BC. The process according to any of embodiments AI-BB, wherein the loading substance has an interfacial tension of less than about 1 dynes/cm.
BD. The process according to any of embodiments AI-BC, wherein the loading substance has an interfacial tension of less than about 0.5 dynes/cm.
BE. The process according to any of embodiments AI-BD, wherein the loading substance comprises algal oil.
BF. The process according to any of embodiments AI-BE, wherein the loading substance comprises oil from a dinoflagellate.
BG. The process according to any of embodiments AI-BF, wherein the loading substance comprises oil from Crypthecodinium cohnii.
BH. The process according to any of embodiments AI-BG, wherein the loading substance comprises fungal oil.
BI. The process according to any of embodiments AI-BH, wherein the loading substance comprises oil from Thraustochytrium, Schizochytrium, or a mixture thereof.
BJ. The process according to any of embodiments AI-BI, wherein the loading substance comprises an omega-3 fatty acid, an alkyl ester of an omega-3 fatty acid, a triglyceride ester of an omega-3 fatty acid, a phytosterol ester of an omega-3 fatty acid,, and/or a mixture thereof.
BK. The process according to any of embodiments AI-BJ, wherein the loading substance comprises docosahexaenoic acid and/or eicosapentaenoic acid, a C1-C6 alkyl ester thereof, a triglyceride ester thereof, a phytosterol ester thereof, and/or a mixture thereof.
BL. The process according to any of embodiments AI-BK, wherein the loading substance is provided in an amount of from about 1 % to about 50% by weight of the aqueous mixture.
BM. The process according to any of embodiments AI-BL, wherein the first polymer component is 240 Bloom fish gelatin, and the pH is from about 9 to about 11.
BN. The process according to any of embodiments AI-BM, wherein the first polymer component is 240 Bloom fish gelatin, and the pH is from about 3.5 to about 4.9.
BO. The process according to any of embodiments AI-BN, wherein the first polymer component is 0 Bloom fish gelatin, and the pH is from about 9 to about 11.
BP. The process according to any of embodiments AI-BO, wherein the first polymer component is 0 Bloom fish gelatin, and the pH is from about 3.5 to about 4.9.
BQ. The process according to any of embodiments AI-BP, wherein the emulsion contains an average droplet size of less than about 1,000 nm.
BR. The process according to any of embodiments AI-BQ, wherein the emulsion contains an average droplet size of less than about 500 nm.
BS. The process according to any of embodiments AI-BR, wherein the emulsion contains an average droplet size of less than about 100 nm.
BT. The process according to any of embodiments AI-BS further comprising adding a surfactant.
BU. An emulsion prepared by the process of any of embodiments AI-BT.
BV. A microcapsule, comprising an agglomeration of primary microcapsules and a loading substance, each individual primary microcapsule having a primary shell, wherein the loading substance comprises an marine oil and has an interfacial tension of less than about 5 dynes/cm, and is encapsulated by the primary shell, and wherein the agglomeration is encapsulated by an outer shell.
BW. The microcapsule according to embodiment BV, wherein the primary shell and/or outer shell comprises a surfactant, gelatin, polyphosphate, polysaccharide, or a mixture thereof.
BX. The microcapsule according to any of embodiments BV-BW, wherein the primary shell and/or outer shell comprises gelatin type B, polyphosphate, gum arabic, alginate, chitosan, carrageenan, pectin, low methoxyl pectin, starch, modified starch, alpha- lactalbumin, beta-lactoglobumin, ovalbumin, polysorbiton, maltodextrin, cyclodextrin, cellulose, methyl cellulose, ethyl cellulose, hydropropylmethylcellulose, carboxymethylcellulose, milk protein, whey protein, soy protein, canola protein, albumin, xanthan, gellan gum, agar, kosher gelatin, non-kosher gelatin, Halal gelatin, non-Halal gelatin, or a mixture thereof.
BY. The microcapsule according to any of embodiments BV-BX, wherein the primary shell and/or outer shell comprise a complex coacervate.
BZ. The microcapsule according to any of embodiments BV-BY, wherein the primary shell and/or outer shell comprise gelatin type A.
CA. The microcapsule according to any of embodiments BV-BZ, wherein the primary shell and/or outer shell comprise fish gelatin.
CB. The microcapsule according to any of embodiments BV-CA, wherein the primary shell and/or outer shell comprise a gelatin with a Bloom number of from about 0 to about 300.
CC. The microcapsule according to any of embodiments BV-CB, wherein the primary shell and/or outer shell comprises a gelatin with a Bloom number of from about 0 to about 50.
CD. The microcapsule according to any of embodiments BV-CC, wherein the primary shell and/or outer shell comprise a gelatin with a Bloom number of from about 51 to about 300.
CE. The microcapsule according to any of embodiments BV-CD, wherein the primary shell and/or outer shell comprise a gelatin with a Bloom number of about 0, about 210, about 220, or about 240.
CF. The microcapsule according to any of embodiments BV-CE, wherein the primary shell and/or outer shell comprise a coacervate of gelatin and polyphosphate.
CG. The microcapsule according to any of embodiments BV-CF, wherein the loading substance has an interfacial tension of less than about 2 dynes/cm.
CH. The microcapsule according to any of embodiments BV-CG, wherein the loading substance has an interfacial tension of less than about 1 dynes/cm.
CI. The microcapsule according to any of embodiments BV-CH, wherein the loading substance has an interfacial tension of less than about 0.5 dynes/cm.
CJ. The microcapsule according to any of embodiments BV-CI, wherein the loading substance comprises algal oil.
CK. The microcapsule according to any of embodiments BV-CJ, wherein the loading substance comprises oil from a dinoflagellate.
CL. The microcapsule according to any of embodiments BV-CK, wherein the loading substance comprises oil from Crypthecodinium cohnii.
CM. The microcapsule according to any of embodiments BV-CL, wherein the loading substance comprises fungal oil.
CN. The microcapsule according to any of embodiments BV-CM, wherein the loading substance comprises oil from Thraustochytrium, Schizochytrium, or a mixture thereof.
CO. The microcapsule according to any of embodiments BV-CN, wherein the loading substance comprises an omega-3 fatty acid, an alkyl ester of an omega-3 fatty acid, a triglyceride ester of an omega-3 fatty acid, a phytosterol ester of an omega-3 fatty acid, and/or a mixture thereof.
CP. The microcapsule according to any of embodiments BV-CO, wherein the loading substance comprises docosahexaenoic acid and/or eicosapentaenoic acid, a C₁-C₆ alkyl ester thereof, a triglyceride ester thereof, a phytosterol ester thereof, and/or a mixture thereof.
CQ. The microcapsule according to any of embodiments BV-CP, wherein the microcapsule has an average diameter of from about 1 µm to about 2,000 µm.
CR. The microcapsule according to any of embodiments BV-CQ wherein the microcapsule has an average diameter of from about 20 µm to about 1,000 µm.
CS. The microcapsule according to any of embodiments BV-CR, wherein the microcapsule has an average diameter of from about 30 µm to about 80 µm.
CT. The microcapsule according to any of embodiments BV-CS, wherein the primary microcapsule has an average diameter of from about 40 nm to about 10 µm.
CU. The microcapsule according to any of embodiments BV-CT, wherein the primary microcapsule has an average diameter of from about 0.1 µm to about 5 µm.
CV. The microcapsule according to any of embodiments BV-CU, wherein the loading substance is from about 20% to about 90% by weight of the microcapsule.
CW. The microcapsule according to any of embodiments BV- CV, wherein the loading substance is from about 50% to about 70% by weight of the microcapsule.
CX. The microcapsule according to any of embodiments BV-CW, wherein the primary shell and outer shell comprise a coacervate of 240 Bloom fish gelatin and sodium polyphosphate.
CY. The microcapsule according to any of embodiments BV-CX, wherein the primary shell and outer shell comprise a coacervate of 0 Bloom fish gelatin and sodium polyphosphate.
CZ. A process for preparing a microcapsule, comprising:
   a. providing an emulsion comprising a first polymer component and a loading substance comprising a marine oil, wherein the emulsion has a pH of greater than about 6.0 or less than about 5.0;
   b. adding a second polymer component to the emulsion;
   c. adjusting pH, temperature, concentration, mixing speed, or a combination thereof to form an aqueous mixture comprising a primary shell material, wherein the primary shell material comprises the first and second polymer components and surrounds the loading substance;
   d. cooling the aqueous mixture to a temperature above the gel point of the primary shell material until the primary shell material forms agglomerations; and
   e. further cooling the aqueous mixture to form an outer shell around the agglomeration.
DA. The process according to embodiment CZ, wherein an antioxidant is added to the emulsion and/or the aqueous mixture.
DB. The process according to any of embodiments CZ-DA, wherein the antioxidant comprises a phenolic compound, a plant extract, or a sulphur-containing compound.
DC. The process according to any of embodiments CZ-DB, wherein the antioxidant comprises ascorbic acid or a salt thereof.
DD. The process according to any of embodiments CZ-DC, wherein the emulsion pH of greater than about 6.0 is achieved by adding sodium hydroxide or wherein the emulsion pH of less than about 5.0 is achieved by adding phosphoric acid.
DE. The process according to any of embodiments CZ-DC, wherein the emulsion pH is from about 3.5 to about 4.9.
DF. The process according to any of embodiments CZ-DE, wherein the emulsion pH is from about 9.0 to about 11.0.
DG. The process according to any of embodiments CZ-DF, wherein the emulsion temperature is initially from about 30°C to about 60°C.
DH. The process according to any of embodiments CZ-DG, wherein the emulsion temperature is initially from about 40°C to about 50°C.
DI. The process according to any of embodiments CZ-DH wherein the emulsion is prepared by emulsifying at from about 1,000 to about 15,000 rpm.
DJ. The process according to any of embodiments CZ-DI, wherein the first polymer component comprises a surfactant, gelatin, polyphosphate, polysaccharide, or a mixture thereof.
DK. The process according to any of embodiments CZ-DJ, wherein the first polymer component comprises gelatin type B, polyphosphate, gum arabic, alginate, chitosan, carrageenan, pectin, low methoxyl pectin, starch, modified starch, alpha- lactalbumin, beta-lactoglobumin, ovalbumin, polysorbiton, maltodextrin, cyclodextrin, cellulose, methyl cellulose, ethyl cellulose, hydropropylmethylcellulose, carboxymethylcellulose, milk protein, whey protein, soy protein, canola protein, albumin, xanthan, gellan gum, agar, kosher gelatin, non-kosher gelatin, Halal gelatin, non-Halal gelatin, or a mixture thereof.
DL. The process according to any of embodiments CZ-DK, wherein the first polymer component comprises gelatin type A.
DM. The process according to any of embodiments CZ-DL, wherein the first polymer component comprises, fish gelatin.
DN. The process according to any of embodiments CZ-DM, wherein the first polymer component has a Bloom number of from about 0 to about 300.
DO. The process according to any of embodiments CZ-DN, wherein the first polymer component has a Bloom number of from about 0 to about 50.
DP. The process according to any of embodiments CZ-DO, wherein the first polymer component has a Bloom number of from about 51 to about 300.
DQ. The process according to any of embodiments CZ-DP, wherein the first polymer component has a Bloom number of about 0, about 210, about 220, or about 240.
DR. The process according to any of embodiments CZ-DQ, wherein the loading substance has an interfacial tension of less than about 5 dynes/cm.
DS. The process according to any of embodiments CZ-DR, wherein the loading substance has an interfacial tension of less than about 2 dynes/cm.
DT. The process according to any of embodiments CZ-DS, wherein the loading substance has an interfacial tension of less than about 1 dynes/cm.
DU. The process according to any of embodiments CZ-DT, wherein the loading substance has an interfacial tension of less than about 0.5 dynes/cm.
DV. The process according to any of embodiments CZ-DU, wherein the loading substance comprises algal oil.
DW. The process according to any of embodiments CV-DV, wherein the loading substance comprises oil from a dinoflagellate.
DX. The process according to any of embodiments CZ-DW, wherein the loading substance comprises oil from Crypthecodinium cohnii.
DY. The process according to any of embodiments CZ-DX, wherein the loading substance comprises fungal oil.
DZ. The process according to any of embodiments CZ-DY, wherein the loading substance comprises oil from Thraustochytrium, Schizochytriumy or a mixture thereof.
EA. The process according to any of embodiments CZ-DZ, wherein the loading substance comprises an omega-3 fatty acid, an alkyl ester of an omega-3 fatty acid, a triglyceride ester of an omega-3 fatty acid, a phyto sterol ester of an omega-3 fatty acid, and/or a mixture thereof.
EB. The process according to any of embodiments CZ-EA, wherein the loading substance comprises docosahexaenoic acid and/or eicosapentaenoic acid, a C₁-C₆ alkyl ester thereof, a triglyceride ester thereof, a phytosterol ester thereof, and/or a mixture thereof.
EC. The process according to any of embodiments CZ-EB, wherein the loading substance is provided in an amount of from about 1% to about 50% by weight of the aqueous mixture.
ED. The process according to any of embodiments CZ-EC, wherein the second polymer component comprises a surfactant, gelatin, polyphosphate, polysaccharide, or a mixture thereof.
EE. The process according to any of embodiments CZ-ED, wherein the second polymer component comprises gelatin type A, gelatin type B, polyphosphate, gum arabic, alginate, chitosan, carrageenan, pectin, low methoxyl pectin, starch, modified starch, alpha-lactalbumin, beta-lactoglobumin, ovalbumin, polysorbiton, maltodextrin, cyclodextrin, cellulose, methyl cellulose, ethyl cellulose, hydropropylmethylcellulose, carboxymethylcellulose, milk protein, whey protein, soy protein, canola protein, albumin, xanthan, gellan gum, agar, kosher gelatin, non-kosher gelatin, Halal gelatin, non-Halal gelatin, or a mixture thereof.
EF. The process according to any of embodiments CZ-EE, wherein the second polymer component is polyphosphate.
EG. The process according to any of embodiments CZ-EF, wherein after step (b), the pH is greater than about 6.0 or less than about 5.0.
EH. The process according to any of embodiments CZ-EG, wherein cooling is at a rate of about 1°C per about 1 to about 100 minutes.
El. The process according to any of embodiments CZ-EH, wherein cooling is at a rate of about 1 °C/5 minute.
EJ. The process according to any of embodiments CZ-EI, wherein the mixture is cooled until it reaches a temperature of from about 5°C to about 10°C.
EK. The process according to any of embodiments CZ-EJ, wherein the mixture is cooled until it reaches a temperature of about 5°C.
EL. The process according to any of embodiments CZ-EK, further comprising step (e) adding a cross-linker to cross-link the shell material.
EM. The process according to any of embodiments CZ-EL, wherein the cross-linker is an enzymatic cross-linker, an aldehyde, tannic acid, alum, or a mixture thereof.
EN. The process according to any of embodiments CZ-EM, wherein the cross-linker is gluteraldehyde.
EO. The process according to any of embodiments CZ-EN, wherein the cross-linker is transglutaminase.
EP. The process according to any of embodiments CZ-EO, further comprising step (f) drying the microcapsules.
EQ. The process according to any of embodiments CZ-EP, wherein the microcapsules are spray dried.
ER. The process according to any of embodiments CZ-EQ, wherein the primary shell and the outer shell comprises a complex coacervate.
ES. The process according to any of embodiments DA-ER, wherein the primary shell and the outer shell comprises a complex coacervate between gelatin and polyphosphate.
ET. The process according to any of embodiments CZ-ES, wherein the microcapsule has an average diameter of from about 1 µm to about 2,000 µm.
EU. The process according to any of embodiments CZ-ET, wherein the microcapsule has an average diameter of from about 20 µm to about 1,000 µm.
EV. The process according to any of embodiments CZ-EU, wherein the microcapsule has an average diameter of from about 30 µm to about 80 µm.
EW. The process according to any of embodiments CZ-EV, wherein the primary microcapsule has an average diameter of from about 40 nm to about 10 µm.
EX. The process according to any of embodiments CZ-EW, wherein the primary microcapsule has an average diameter of from about 0.1 µm to about 5 µm.
EY. The process according to any of embodiments CZ-EX, wherein the loading substance is from 20% to 90% by weight of the microcapsule.
EZ. The process according to any of embodiments CZ-EY, wherein the loading substance is from 50% to 70% by weight of the microcapsule.
FA. The process according to any of embodiments CZ-EZ, wherein the first polymer component is 240 Bloom fish gelatin, the second polymer component is sodium polyphosphate and the emulsion pH is from about 9 to about 11.
FB. The process according to any of embodiments CZ-FA, wherein the first polymer component is 240 Bloom fish gelatin, the second polymer component is sodium polyphosphate and the emulsion pH is from about 3.5 to about 4.9.
FC. The process according to any of embodiments CZ-FB, wherein the first polymer component is 0 Bloom fish gelatin, the second polymer component is sodium polyphosphate and the emulsion pH is from about 9 to about 11.
FD. The process according to any of embodiments CZ-FC, wherein the first polymer component is 0 Bloom fish gelatin, the second polymer component is sodium polyphosphate and the emulsion pH is from about 3.5 to about 4.9.
FE. A microcapsule prepared according to the process of any of embodiments CZ-FD.
FF. A method of delivering a loading substance to a subject, comprising administering to the subject a microcapsule of any of embodiments BV-CY and FE and/or an emulsion of any of embodiments A-AD and BU.
FG. The method of embodiment FF, wherein the subject is a mammal.
FH. The method of embodiment FF, wherein the subject is a human.
FI. A use of a microcapsule of any of embodiments BV-CY and FE to prepare a medicament for delivering a loading substance to a subject.
FJ. A use of an emulsion of any of embodiments A-AD and BU to prepare a medicament for delivering a loading substance to a subject.
FK. A formulation vehicle comprising a microcapsule of any of embodiments BV-CY and FE and/or an emulsion of any of embodiments A-AD and BU.
FL. The formulation vehicle of embodiment FK, wherein the formulation vehicle is a foodstuff, a beverage, a nutraceutical formulation,. or a pharmaceutical formulation.

## Claims

1. An emulsion, comprising a gelatin and a loading substance, wherein the loading substance comprises microbial oil and has an interfacial tension of less than 5 dynes/cm and wherein the emulsion has a pH of from 3.5 to 4.9.

2. The emulsion according to claim 1, further comprising an antioxidant and/or a surfactant and/or sodium hydroxide, phosphoric acid, or a mixture thereof.

3. The emulsion according to claim 2, wherein the antioxidant comprises a phenolic compound, a plant extract, or a sulphur-containing compound, or ascorbic acid or a salt thereof.

4. The emulsion according to any of the preceding claims further comprising a surfactant, polyphosphate, polysaccharide, or a mixture thereof;
gum arabic, alginate, chitosan, carrageenan, pectin, low methoxyl pectin, starch, modified starch, alpha- lactalbumin, beta-lactoglobumin, ovalbumin, polysorbiton, maltodextrin, cyclodextrin, cellulose, methyl cellulose, ethyl cellulose, hydropropylmethylcellulose, carboxymethylcellulose, milk protein, whey protein, soy protein, canola protein, albumin, xanthan, gellan gum, agar, or a mixture thereof.

5. The emulsion according to any of the preceding claims, wherein the gelatin comprises gelatin type B, gelatin type A, kosher gelatin, non-kosher gelatin, Halal gelatin, non-Halal gelatin, or a mixture thereof.

6. The emulsion according to any of the preceding claims, wherein the gelatin has a Bloom number of from 0 to 300, preferably 0 to 50 or 51 to 300, e.g. 0, 210, 220, or 240.

7. The emulsion according to any of the preceding claims, wherein the loading substance comprises an algal oil, oil from a dinoflagellate, oil from *Crypthecodinium cohnii,* fungal oil, oil from *Thraustochytrium, Schizochytrium,* or a mixture thereof, an omega-3 fatty acid, an alkyl ester of an omega-3 fatty acid, a triglyceride ester of an omega-3 fatty acid, a phytosterol ester of an omega-3 fatty acid, and/or a mixture thereof, docosahexaenoic acid and/or eicosapentaenoic acid, a C₁-C₆ alkyl ester thereof, a triglyceride ester thereof, a phytosterol ester thereof, and/or a mixture thereof.

8. The emulsion according to any of the preceding claims, wherein the loading substance is from 1% to 50% by weight of the emulsion.

9. The emulsion according to any of the preceding claims, wherein the gelatin is 240 Bloom fish gelatin, the loading substance is one or more microbial oils or mixtures thereof, and the pH is from 3.5 to 4.9 or wherein the first polymer component is 0 Bloom fish gelatin and the pH is from 3.5 to 4.9.

10. An emulsion of any of claims 1 to 9 for use in preparing a medicament for delivering a loading substance to a subject.

11. A formulation vehicle comprising an emulsion of any of claims 1-9, preferably wherein the formulation vehicle is a foodstuff, a beverage, a nutraceutical formulation, or a pharmaceutical formulation.

12. A process for preparing an emulsion, comprising providing an aqueous mixture of gelatin and a loading substance and optionally an antioxidant and/or a surfactant and/or sodium hydroxide, phosphoric acid, or a mixture thereof;
wherein the loading substance comprises a marine oil that has an interfacial tension of less than about 5 dynes/cm and wherein the mixture has a pH of from 3.5 to 4.9; and emulsifying the mixture.

13. The process according to claim 12, wherein emulsifying is performed at a temperature of from 30°C to 60°C and/or wherein the aqueous mixture is emulsified at from 1,000 to 15,000 rpm.

14. The process according to claim 12 or 13, further comprising dehydrating the emulsion.

15. The process according to claim 12 to 14, wherein the emulsion contains an average droplet size of less than 1,000 nm, preferably less than 500 nm, or more preferably less than 100 nm.

16. The process according to claim 12 to 15, wherein the loading substance comprises an algal oil, oil from a dinoflagellate, oil from *Crypthecodiniwn cohnii,* fungal oil, oil from *Thraustochytrium, Schizochytrium,* or a mixture thereof, an omega-3 fatty acid, an alkyl ester of an omega-3 fatty acid, a triglyceride ester of an omega-3 fatty acid, a phytosterol ester of an omega-3 fatty acid, and/or a mixture thereof, docosahexaenoic acid and/or eicosapentaenoic acid, a C₁-C₆ alkyl ester thereof, a triglyceride ester thereof, a phytosterol ester thereof, and/or a mixture thereof.

17. The process according to claim 12 to 16, wherein the loading substance is from 1% to 50% by weight of the emulsion.

18. A microcapsule, comprising an agglomeration of primary microcapsules and a loading substance, each individual primary microcapsule having a primary shell, wherein the loading substance comprises an marine oil and has an interfacial tension of less than about 5 dynes/cm, and is encapsulated by the primary shell, and wherein the agglomeration is encapsulated by an outer shell.
